# EUROPEAN PATENT SPECIFICATION

(11) **EP 3 393 500 B1**
(45) Date of publication and mention of the grant of the patent: **13.10.2021**
(21) Application number: 16880062.1
(22) Date of filing: 21.12.2016
(51) Int. Cl.: A61K 47/60, A61K 47/58, A61K 47/59, A61K 47/65, C12N 9/06

(54) **POLYMER CONJUGATES HAVING REDUCED ANTIGENICITY**
POLYMERKONJUGATE MIT VERRINGERTER ANTIGENIZITÄT
CONJUGUÉS POLYMÈRES AYANT UNE ANTIGÉNICITÉ RÉDUITE

(30) Priority: 21.12.2015 US 201562270401 P; 18.03.2016 US 201662310534 P; 29.04.2016 US 201662329800 P; 12.10.2016 US 201662407403 P
(43) Date of publication of application: 31.10.2018
(73) Proprietor: Duke University, Durham, NC 27705 (US)
(72) Inventor: GANSON, Nancy J., Durham,NC 27705 (US); CHILKOTI, Ashutosh, Durham,NC 27701 (US); QI, Yizhi, Durham,NC 27705 (US); HERSHFIELD, Michael S., Durham,NC 27705 (US)
(74) Representative: Chapman, Paul Gilmour
(86) International application number: PCT/US2016/068141
(87) International publication number: WO 2017/112825

(56) References cited:
- US-A1- 2002 052 443
- US-A1- 2004 053 976
- GAO WEIPING ED - HANES JUSTIN ET AL: "Site-specific andin situgrowth of stealth polymer conjugates of proteins with significantly improved pharmacology", JOURNAL OF CONTROLLED RELEASE, vol. 172, no. 1, 28 November 2013 (2013-11-28), XP028772957, ISSN: 0168-3659, DOI: 10.1016/J.JCONREL.2013.08.279
- W. GAO ET AL: "In situ growth of a PEG-like polymer from the C terminus of an intein fusion protein improves pharmacokinetics and tumor accumulation", PNAS, vol. 107, no. 38, 21 September 2010 (2010-09-21), pages 16432-16437, XP055297344, US ISSN: 0027-8424, DOI: 10.1073/pnas.1006044107
- VIEGAS ET AL.: 'Polyoxazoline: Chemistry, Properties, and Applications in Drug Delivery' BIOCONJUGATE CHEM. vol. 22, 24 January 2011, pages 976 - 986, XP055082338
- TONG ET AL.: 'Protein Modification with Amphiphilic Block Copoly(2-oxazoline)s as a New Platform for Enhanced Cellular Delivery' MOL PHARM vol. 7, no. 4, 02 August 2010, pages 984 - 992, XP055409443
- KHANDARE ET AL.: 'Polymer drug conjugates: Progress in polymeric prodrugs' PROG. POLYM. SCI. vol. 31, 12 September 2005, pages 359 - 397, XP027932362
- QI ET AL.: 'Protein-Polymer Conjugation?Moving Beyond PEGylation' CURR OPIN CHEM BIOL vol. 28, 31 October 2015, pages 181 - 193, XP055409460
- ODAY ET AL.: 'Therapeutic Protein Polymer Conjugates: Advancing Beyond PEGylation' J. AM. CHEM. SOC. vol. 136, 12 September 2014, pages 14323 - 14332, XP055409454

## Description

### FIELD

This disclosure relates to molecule-polymer conjugates having reduced or eliminated antigenicity.

### INTRODUCTION

With more than a hundred peptides and proteins approved by the FDA to treat various diseases and many more in clinical and pre-clinical development, therapeutic peptides and proteins are an important class of drugs today. However, the clinical use of peptides and proteins is often challenged by their short plasma half-life, which can necessitate frequent injections and cause an undesirable peak-to-valley fluctuation of the drug concentration *in vivo* as well as reduce patient compliance and increase treatment cost. Other limitations of peptide and protein therapeutics may include poor stability, low solubility and immunogenicity. To address these limitations, various delivery strategies have been developed for sustained delivery of peptide and protein therapeutics, ranging from particulate systems, depots, to chemical conjugation with long circulating polymers such as poly(ethylene glycol) (PEG), or recombinant fusions with long circulating proteins such as albumin or the Fc domain of antibodies.

PEGylation, or the covalent conjugation of therapeutics with the "stealth" polymer PEG, is one of the most widely used approaches to increase the circulation half-life and stability and to reduce the immunogenicity of biomolecule therapeutics such as polypeptides and polynucleotides. However, after nearly four decades of research and over two decades of clinical use, the drawbacks of PEGylation have begun to emerge. Conventional methods for the synthesis of PEGylated conjugates have significant limitations: (1) conjugation involves the reaction between protein-repulsive PEG chains and biomacromolecules, so that even with a large excess of polymer, steric hindrance still results in a low yield of conjugate, typically in the 10-20% range; (2) the presence of a large excess of unreacted polymer makes product purification non-trivial; and (3) conjugation typically involves reacting the chain-ends of the polymer with reactive side-groups on lysine and cysteine residues, which are often promiscuously distributed on the biomolecule, thus yielding chemically heterogeneous products that can significantly compromise the bioactivity of the drug and greatly complicate regulatory approval.

Furthermore, the immunogenicity of PEG has recently attracted much attention. Anti-PEG antibodies have been induced in patients treated with some PEGylated enzymes, and in clinical trials of PEG-uricase and PEG-asparaginase, these anti-PEG antibodies have markedly accelerated blood clearance, abrogated clinical efficacy, and increased the risk and severity of infusion reactions. Circulating anti-PEG antibodies have also been found in individuals naïve to PEGylated materials, possibly induced by chronic exposure to free PEGs present in commonly used consumer products. High levels of such pre-existing anti-PEG antibodies have recently been linked to serious first-exposure allergic reactions to a PEGylated RNA aptamer, which led to early termination of a clinical trial.

Gao et al, Journal of Controlled Release Vol.172(1) 28 November 2013 e116-e117 (XP028772957) describes stealth polymer conjugates. Gao et al PNAS Vol.107(38) 21 September 2010 16432-16437 (XP055297344) describes methods of growing PEG-like polymers. There is a need for modifying biomolecule therapeutics to increase their circulation half-life and stability and to reduce their antigenicity or ability to bind pre-existing antibodies.

### SUMMARY

In an aspect, the disclosure relates to methods of reducing the antigenicity of a molecule. The methods include conjugating at least one branched polymer to a molecule to form a molecule-polymer conjugate, wherein the molecule comprises a polypeptide, a polynucleotide, a small molecule, or a combination thereof, wherein the branched polymer comprises poly[oligo(ethylene glycol) methyl ether methacrylate] (POEGMA), wherein the POEGMA comprises a backbone comprising poly(methyl methacrylate) and a plurality of side chains, each side chain is covalently attached to the backbone, where each side chain comprises 2 to 7 monomers of ethylene glycol (EG) repeated in tandem. The molecule-polymer conjugate is not reactive with pre-existing anti-PEG antibodies in a subject, and the molecule-polymer conjugate has reduced or eliminated antigenicity compared to a control comprising the molecule conjugated to the unbranched PEG. In some embodiments, the molecule is conjugated to the backbone of the branched polymer. In some embodiments, the molecule is conjugated to the backbone of the branched polymer via a linker. In some embodiments, each side chain has a first terminal end and a second terminal end, wherein the first terminal end is covalently attached to the backbone, and wherein the second terminal end independently comprises an alkyl, ester, amine, amide, or carboxyl group. In some embodiments, each side chain has a first terminal end and a second terminal end, wherein the first terminal end is covalently attached to the backbone, and wherein the second terminal end does not include a hydroxyl group. In some embodiments, each side chain comprises 3 to 7 monomers repeated in tandem. In some embodiments, each side chain comprises 3 monomers repeated in tandem. In some embodiments, more than one branched polymer is conjugated to the molecule, each branched polymer conjugated to a different site of the molecule. In some embodiments, the molecule comprises a polypeptide, and wherein one branched polymer is conjugated to the polypeptide at a site selected from the C-terminus, the N-terminus, and an internal amino acid of the polypeptide. In some embodiments, the molecule comprises a polypeptide, and wherein more than one branched polymer is conjugated to the polypeptide, each branched polymer conjugated to a different site of the polypeptide selected from the C-terminus, the N-terminus, an internal amino acid, or a combination thereof.

In some embodiments, the molecule comprises a polypeptide comprising a sortase A recognition site, and wherein the branched polymer and the polypeptide are incubated with sortase A under conditions to conjugate the branched polymer to the sortase recognition site of the polypeptide. In some embodiments, the molecule comprises a polypeptide comprising a sortase A recognition site, and wherein the conjugating comprises: a) contacting the molecule with a sortase A and an initiator agent under conditions that permit attachment of the initiator agent to the sortase A recognition site to form a macroinitiator; and b) incubating the macroinitiator with a monomer under conditions that permit free-radical polymerization and formation of a branched polymer to occur from the initiator agent to form the molecule-polymer conjugate. In some embodiments, the sortase A recognition site comprises LPXTG (SEQ ID NO: 1), wherein X is any amino acid. In some embodiments, the macroinitiator and monomer are incubated with a catalyst in step (b). In some instances, which are not presently claimed, the monomer in step (b) comprises at least one of an acrylate, methacrylate, acrylamide, and methacrylamide. In some embodiments, the method further includes separating the molecule-polymer conjugate formed in step (b) from the unreacted macroinitiator. In some embodiments, the branched polymer is synthesized and subsequently grafted to the molecule to form the molecule-polymer conjugate. In some embodiments, the conjugating comprises attaching an initiator agent to the molecule to form a macroinitiator; and incubating the macroinitiator with a monomer under conditions that permit free-radical polymerization and formation of a branched polymer to occur from the initiator agent to form the molecule-polymer conjugate. In some embodiments, the branched polymer is synthesized using free-radical polymerization. In some embodiments, the branched polymer is synthesized using at least one method selected from ionic ring-opening polymerization (ionic ROP), ring opening metathesis polymerization, ionic polymerization, condensation polymerization, and coordination polymerization.

Also described herein, but not presently claimed are methods of making a molecule-polymer conjugate having reduced or eliminated antigenicity compared to a control, from a molecule comprising a polypeptide having a sortase A recognition site. The method may include a) contacting the molecule with a sortase A and an initiator agent under conditions that permit attachment of the initiator agent to the sortase A recognition site to form a macroinitiator; and b) incubating the macroinitiator with a monomer under conditions that permit free-radical polymerization and formation of a branched polymer to occur from the initiator agent to form the molecule-polymer conjugate, wherein the branched polymer comprises a backbone and a plurality of side chains, each side chain covalently attached to the backbone. The sortase A recognition site may comprise LPXTG (SEQ ID NO: 1), wherein X is any amino acid. The macroinitiator and monomer may be incubated with a catalyst in step (b). The monomer in step (b) may comprise at least one of an acrylate, methacrylate, acrylamide, and methacrylamide. The method may further include separating the molecule-polymer conjugate formed in step (b) from the unreacted macroinitiator, wherein the yield of molecule-polymer conjugate is at least about 50% of the total conjugates and macroinitiators which are separated. The molecule-polymer conjugate may be separated by chromatography. The chromatography may comprise size-exclusion chromatography, ion exchange chromatography, affinity chromatography, or hydrophobic interaction chromatography, or a combination thereof. The chromatography may comprise size-exclusion chromatography. The free-radical polymerization may comprise at least one of atom transfer radical polymerization (ATRP), reversible addition-fragmentation chain transfer (RAFT), radical ring-opening polymerization (radical ROP), nitroxide-mediated radical polymerization (NMP), iniferter polymerization, free radical polymerization, cobalt-mediated radical polymerization, telluride-mediated polymerization, and stibine-mediated polymerization. The molecule may comprise a polypeptide, a polynucleotide, a small molecule, or a combination thereof.

Another aspect of the disclosure provides a molecule-polymer conjugate having reduced or eliminated antigenicity compared to a control. The molecule-polymer conjugates include a branched polymer comprising poly[oligo(ethylene glycol) methyl ether methacrylate] (POEGMA). The POEGMA comprises a backbone comprising poly(methyl methacrylate) and a plurality of side chains covalently attached to the backbone where each side chain comprises 2 to 7 monomers of ethylene glycol (EG) repeated in tandem; wherein the control comprises the molecule conjugated to unbranched PEG; and wherein the molecule-polymer conjugate is not reactive with pre-existing anti-PEG antibodies in a subject. The molecule-polymer conjugate also comprises a molecule conjugated to the backbone of the branched polymer, wherein the molecule comprises a polypeptide, a polynucleotide, a small molecule, or a combination thereof. In some embodiments, the molecule is conjugated to the backbone of the branched polymer via a linker. In some embodiments, each side chain has a first terminal end and a second terminal end, wherein the first terminal end is covalently attached to the backbone, and wherein the second terminal end independently comprises an alkyl, ester, amine, amide, or carboxyl group. In some embodiments, each side chain has a first terminal end and a second terminal end, wherein the first terminal end is covalently attached to the backbone, and wherein the second terminal end does not include a hydroxyl group. In some embodiments, each side chain comprises 3 to 7 monomers repeated in tandem. In some embodiments, each side chain comprises 3 monomers repeated in tandem. In some embodiments, more than one branched polymer is conjugated to the molecule, each branched polymer conjugated to a different site of the molecule. In some embodiments, the molecule comprises a polypeptide, and wherein one branched polymer is conjugated to the polypeptide at a site selected from the C-terminus, the N-terminus, and an internal amino acid of the polypeptide. In some embodiments, the molecule comprises a polypeptide, and wherein more than one branched polymer is conjugated to the polypeptide, each branched polymer conjugated to a different site of the polypeptide selected from the C-terminus, the N-terminus, an internal amino acid, or a combination thereof. The branched polymer comprises poly[oligo(ethylene glycol) methyl ether methacrylate] (POEGMA), and wherein the POEGMA comprises: a backbone comprising poly(methyl methacrylate); and a plurality of side chains covalently attached to the backbone, each side chain comprising 2 to 7 monomers of ethylene glycol (EG) repeated in tandem. In some embodiments, each side chain comprises 3 monomers of ethylene glycol (EG) repeated in tandem. In some embodiments, each side chain comprises 3 to 7 monomers of ethylene glycol (EG) repeated in tandem. The molecule-POEGMA conjugate is not reactive with pre-existing anti-PEG antibodies in a subject.

In some embodiments, the molecule comprises one or more peptides or protein therapeutic agents selected from a monoclonal antibody, blood factor, betatrophin, exendin, enzyme, asparaginase, glutamase, arginase, arginine deaminase, adenosine deaminase (ADA), ADA-2, ribonuclease, cytosine deaminase, trypsin, chymotrypsin, papain, growth factor, epidermal growth factor (EGF), insulin, insulin-like growth factor (IGF), transforming growth factor (TGF), nerve growth factor (NGF), platelet-derived growth factor (PDGF), bone morphogenic protein (BMP), fibroblast growth factor (FGF), somatostatin, somatotropin, somatropin, somatrem, calcitonin, parathyroid hormone, colony stimulating factors (CSF), clotting factors, tumor necrosis factors (TNF), gastrointestinal peptides, vasoactive intestinal peptide (VIP), cholecystokinin (CCK), gastrin, secretin, erythropoietins, growth hormone , GRF, vasopressins, octreotide, pancreatic enzymes, superoxide dismutase, thyrotropin releasing hormone (TRH), thyroid stimulating hormone, luteinizing hormone, luteinizing hormone-releasing hormone (LHRH), growth hormone releasing hormone (GHRH), tissue plasminogen activators, interleukins, interleukin-1, interleukin-15, interleukin-2, interleukin-10, colony stimulating factor, granulocyte macrophage colony-stimulating factor (GM-CSF), interleukin-1 receptor antagonist (IL-1RA), glucagon-like peptide-1 (GLP-1), exenatide, GLP-1 R multi-agonist, GLP-1 R antagonist, GLP-2, TNF-related apoptosis-inducing ligand (TRAIL), leptin, ghrelin, granulocyte monocyte colony stimulating factor (GM-CSF), interferons, interferon-a, interferon-gamma, human growth hormone (hGH) and antagonist, macrophage activator, chorionic gonadotropin, heparin, atrial natriuretic peptide, hemoglobin, relaxin, cyclosporine, oxytocin, vaccines, monoclonal antibodies, single chain antibodies, ankyrin repeat proteins, affibodies, activin receptor 2A extracellular domain, alpha-2 macroglobulin, alpha-melanocyte, apelin, bradykinin B2 receptor antagonist, cytotoxic T-lymphocyte-associated protein (CTLA-4), elafin, Factor IX, Factor VIIa, Factor VIII, hepcidin, infestin-4, kallikrein inhibitor, L4F peptide, lacritin, parathyroid hormone (PTH), peptide YY (PYY), thioredoxin, thymosin B4, urate oxidase, urodilatin, aptamers, silencing RNA, microRNA, long non-coding RNA, ribozymes, analogs and derivatives thereof, and combinations thereof. In some embodiments, the molecule comprises a polypeptide, and wherein the polypeptide comprises a His-tag, a stimulus-responsive polypeptide, or a combination thereof. In some embodiments, the stimulus-responsive polypeptide is selected from an elastin-like polypeptide, a polypeptide comprising a repeated motif, and a resilin-like polypeptide. In some embodiments, the molecule-polymer conjugate has: an *in vivo* half-life that is at least 25% greater compared with the *in vivo* half-life of the molecule itself; or an *in vivo* biodistribution to a tissue, organ, or disease site that is at least 25% greater than the *in vivo* biodistribution of the molecule itself; or a reduced binding to anti-PEG antibodies compared to a control; or a reduced immune response compared to a control; or a combination thereof. In some embodiments, the molecule-polymer conjugates have an *in vivo* half-life that is at least 80% greater than the *in vivo* half-life of the molecule itself. According to the present claims, the control comprises the molecule conjugated to unbranched PEG. In some embodiments, the molecule comprises a polypeptide, and wherein at least about 20% of the polypeptides have a conjugated branched polymer solely at the C-terminus. In some embodiments, at least about 75% of the polypeptides have a conjugated branched polymer solely at the C-terminus. In some embodiments, at least about 90% of the polypeptides have a conjugated branched polymer solely at the C-terminus. In some embodiments, the yield of molecule-polymer conjugate is at least about 75%. In some embodiments, the yield of molecule-polymer conjugate is at least about 85%.

The disclosure provides for other aspects and embodiments that will be apparent in light of the following detailed description and accompanying figures.

### BRIEF DESCRIPTION OF THE DRAWINGS

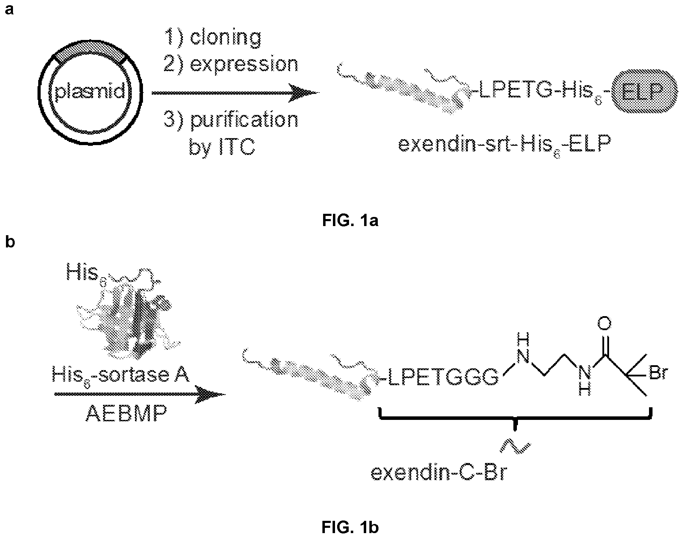

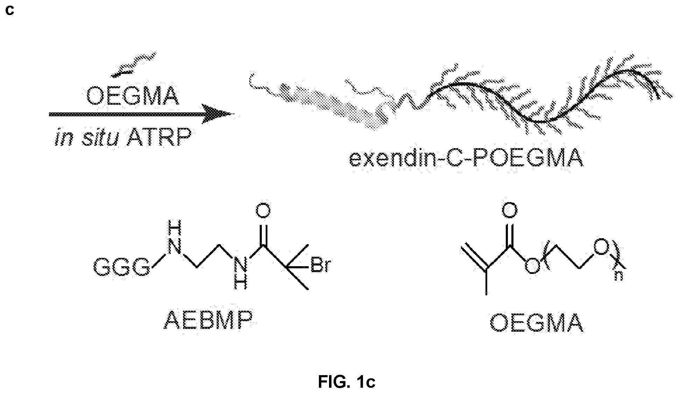

**FIG. 1****.** Synthetic scheme of exendin-C-POEGMA. **a**) Recombinant expression of the sortase A substrate, exendin-srt-His₆-ELP, and purification by ITC. **b**) Sortase-catalyzed site-specific attachment of the ATRP initiator AEBMP to the C-terminus of exendin to generate exendin-C-Br. **c**) *In situ* ATRP of OEGMA from exendin-C-Br yielding exendin-C-POEGMA. ITC: inverse transition cycling, ELP: elastin-like polypeptide, srt: sortase A recognition sequence "LPETG" (SEQ ID NO: 2), AEBMP: N-(2-(2-(2-(2-aminoacetamido)acet-amido)acetamido) ethyl)-2-bromo-2-methylpropanamide. Images from the RCSB PDB (www.rcsb.org) of: PDB ID 1T2P (sortase A); PDB ID 1JRJ (exendin-4).

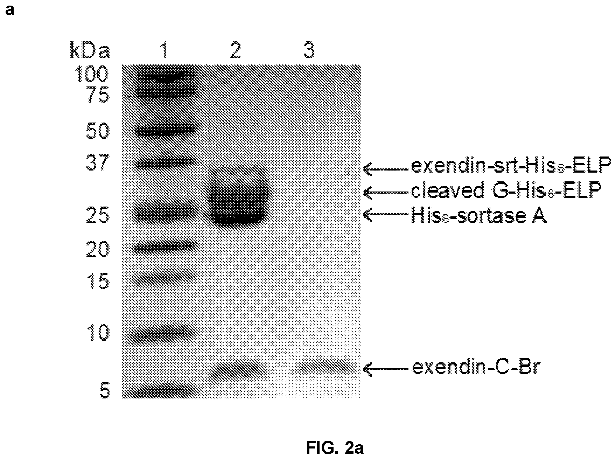

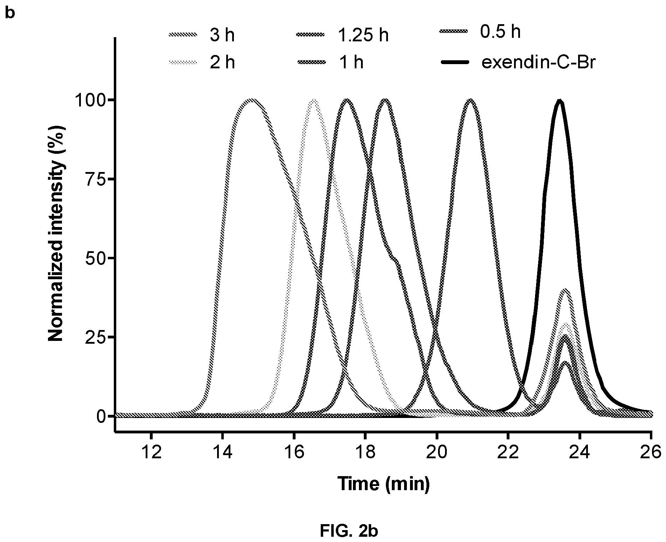

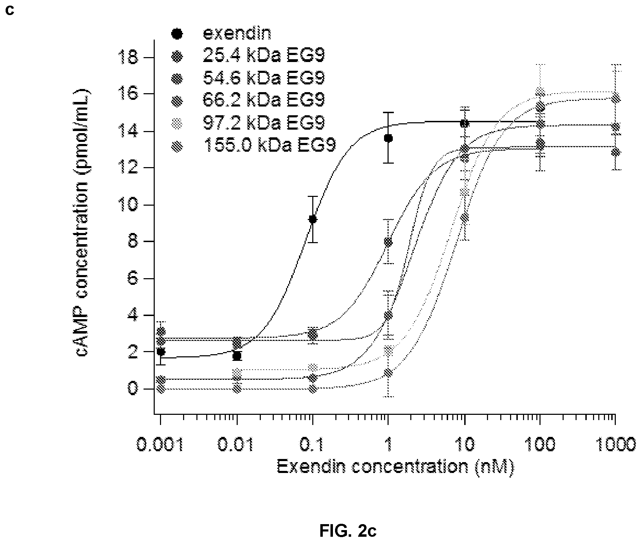

**FIG. 2****.** Characterization of exendin-C-Br macroinitiator and EG9 exendin-C-POEGMA conjugates. **a**) Coomassie-stained SDS-PAGE analysis of initiator attachment on exendin by sortase A. Lane 1: MW marker, lane 2: sortase reaction mixture after 18 h of reaction, lane 3: purified exendin-C-Br macroinitiator. **b**) SEC traces of ATRP reaction mixtures of grafting EG9 POEGMA from exendin-C-Br carried out for 0.5 h, 1 h, 1.25 h, 2 h and 3 h, detected by UV-vis absorbance at 280 nm. **c**) Cyclic adenosine monophosphate (cAMP) response of native exendin and EG9 exendin-C-POEGMA conjugates with Mₙs of 25.4 kDa, 54.6 kDa, 66.2 kDa, 97.2 kDa and 155.0 kDa in baby hamster kidney (BHK) cells expressing the GLP-1R. Results are plotted as mean ± standard error of the mean (SEM), n=3. Half-maximal effective concentration (EC₅₀) values are summarized in **TABLE 1.**

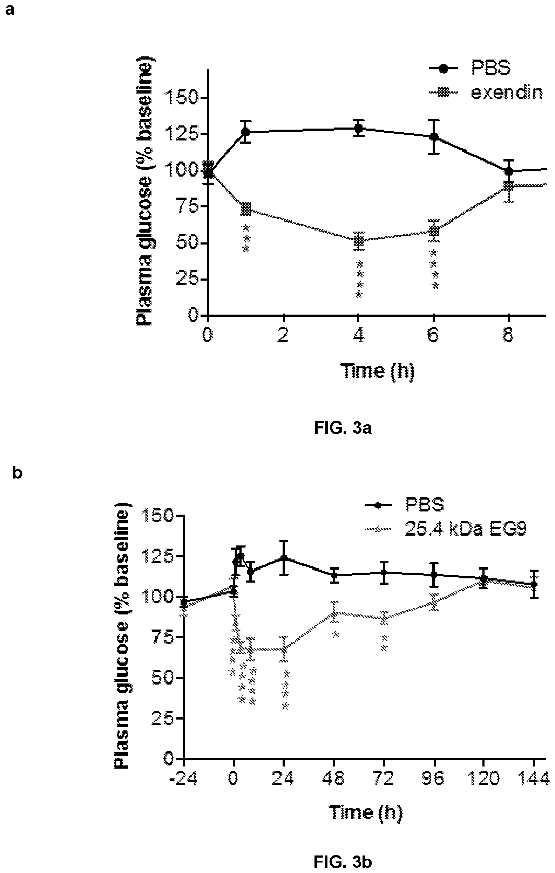

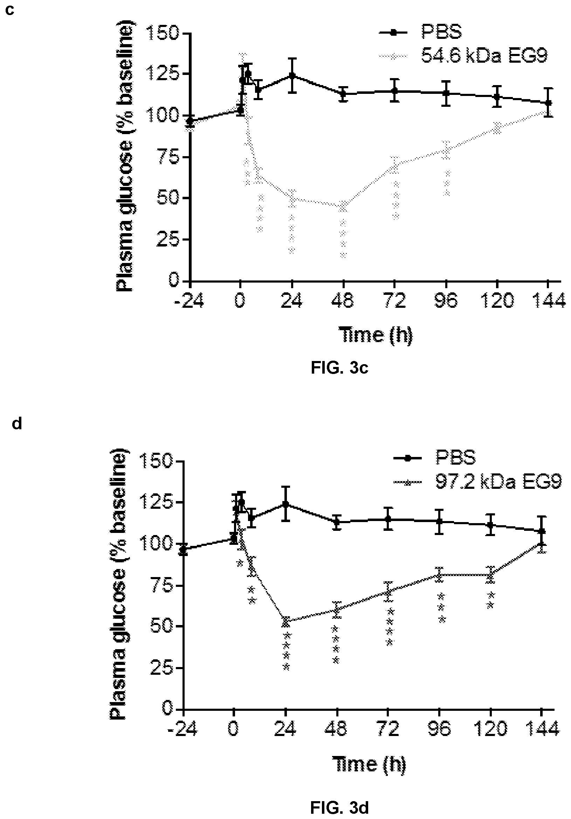

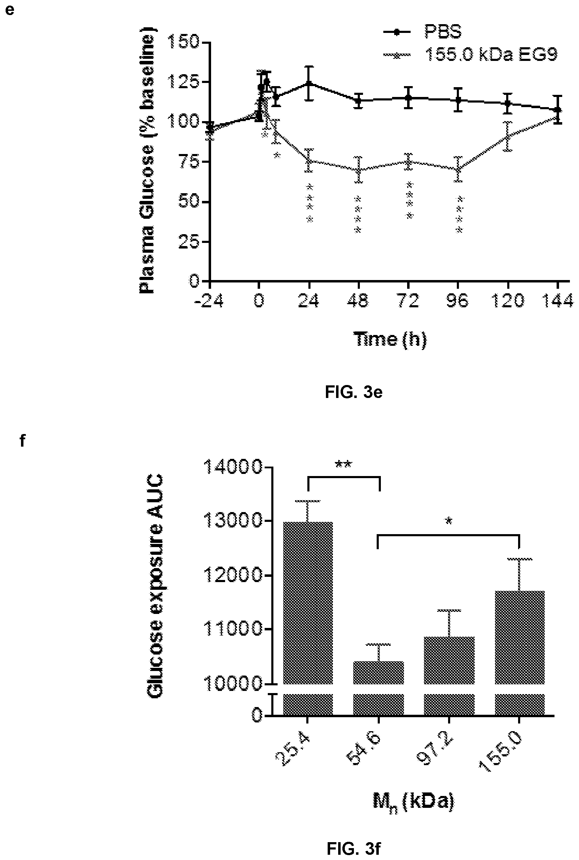

**FIG. 3****.** Assessment of MW-dependent *in vivo* efficacy of EG9 exendin-C-POEGMA conjugates. Blood glucose levels in fed mice were measured before and after a single s.c. injection of **a**) unmodified exendin, or **b-e**) 25.4 kDa, 54.6 kDa, 97.2 kDa, and 155.0 kDa EG9 exendin-C-POEGMA conjugates, compared to PBS control. The peptide and conjugates were administered at 25 nmol/kg and PBS was injected at equivalent volume at t = 0 h. Blood glucose levels were normalized to the average glucose levels measured 24 h and immediately before injection. Data were analyzed by repeated measures two-way analysis of variance (ANOVA), followed by *post hoc* Dunnett's multiple comparison test. **f**) Area under the curve (AUC) of blood glucose profiles (0 h to 144 h, with respect to 0% baseline) as a function of conjugate Mₙ. AUCs were compared using one-way ANOVA followed by *post hoc* Tukey's multiple comparison test. In all panels, results are plotted as mean ± SEM, n=6, **P* < 0.05, ***P* < 0.01, ****P* < 0.001 and *****P* <0.0001.

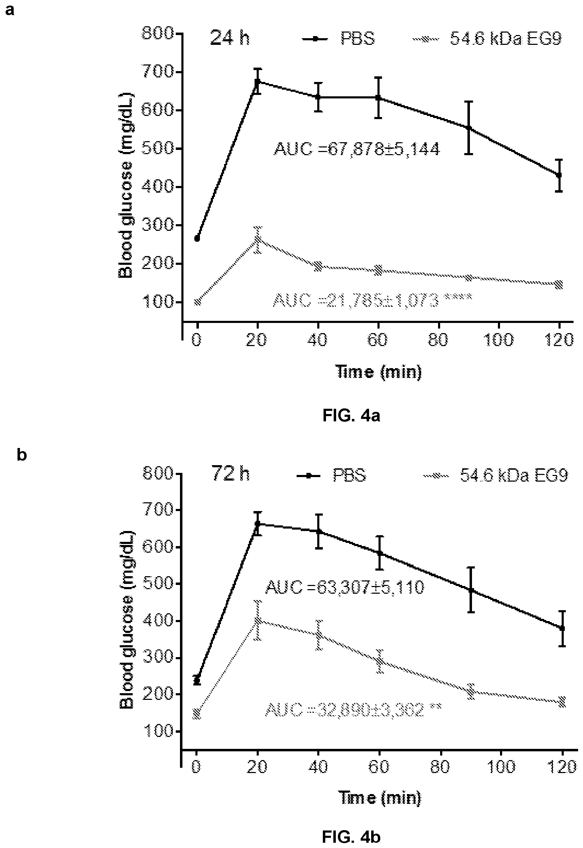

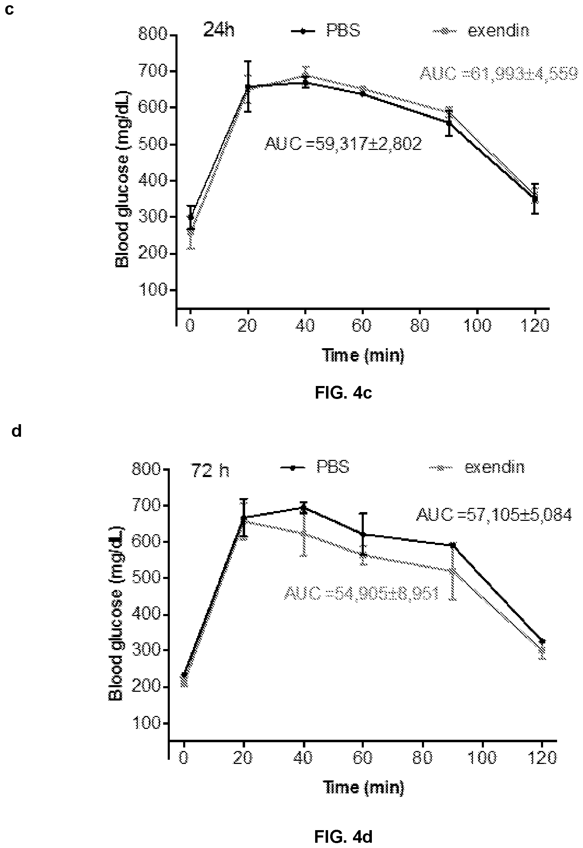

**FIG. 4****.** Intraperitoneal glucose tolerance test (IPGTT) of an EG9 exendin-POEGMA in mice. Mouse blood glucose levels measured in an IPGTT performed at 24 h and 72 h after a single s.c. injection of **a**) and **b**) the 54.6 kDa EG9 exendin-POEGMA conjugate or **c**) and **d**) unmodified exendin at 25 nmol/kg, compared to PBS of equivalent volume. Mice were fasted for 6 h prior to glucose challenge by an intraperotoneal (i.p.) injection of 1.5 g/kg of glucose. Results are plotted as mean ± SEM, n=5 in panels **a**) and **b**), n=3 in panels c and d. AUCs of treatment and PBS were compared using an unpaired parametric two-tailed t test (***P* < 0.01, and *****P* <0.0001). Exendin was not significant at either time point.

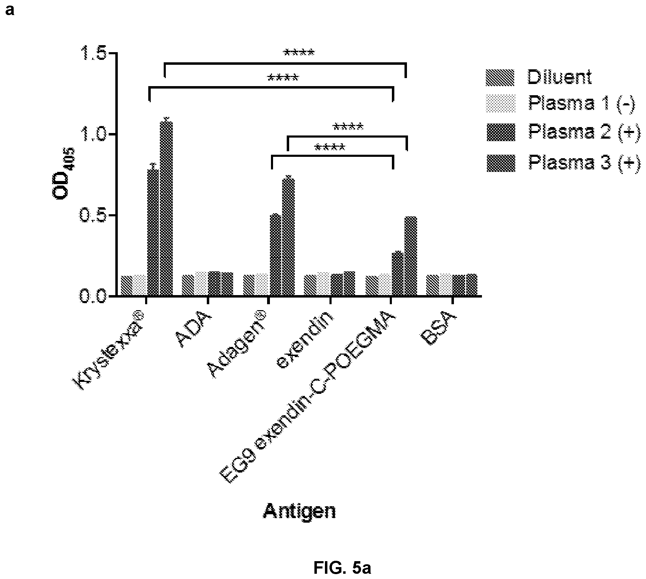

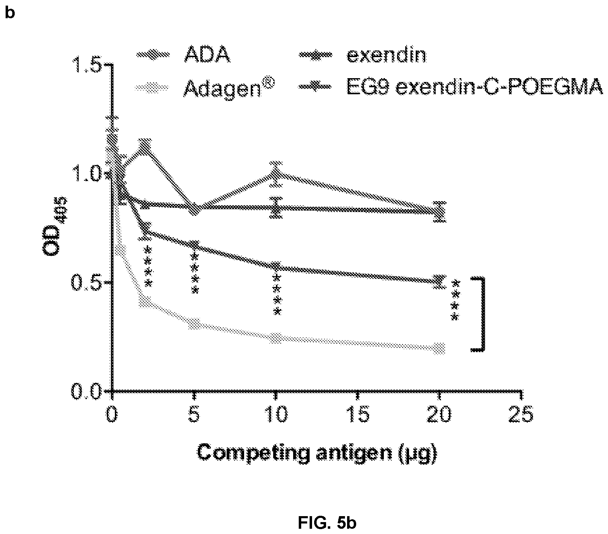

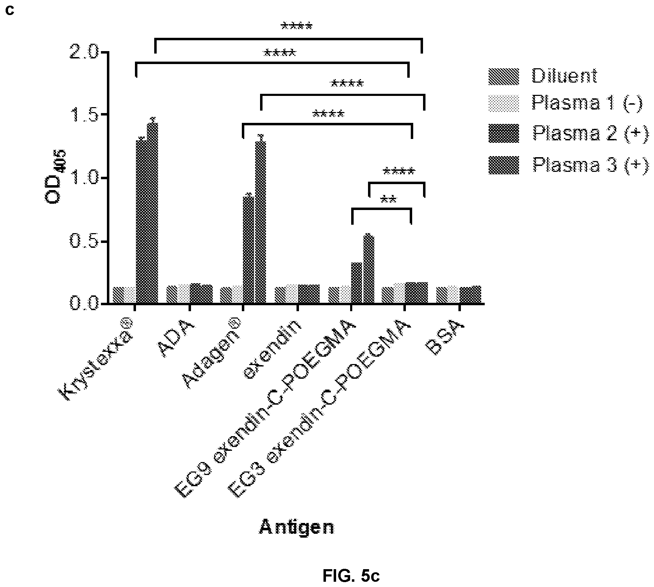

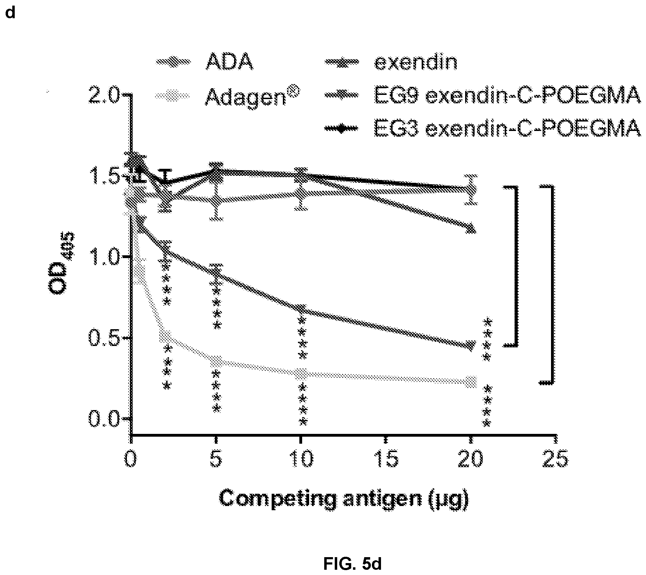

**FIG. 5**. Assessment of reactivity of exendin-C-POEGMA conjugates toward anti-PEG antibodies in patient plasma samples. **a**) Direct ELISA probing 54.6 kDa EG9 exendin-C-POEGMA conjugate, native exendin, adenosine deaminase (ADA), bovine serum albumin (BSA), Krystexxa^{®} (PEG-uricase) and Adagen^{®} (PEG-ADA) with diluent (1% BSA in PBS), an anti-PEG negative patient plasma sample, or one of two anti-PEG positive plasma samples. **b**) Competitive ELISA, where various amounts of exendin, 54.6 kDa EG9 exendin-C-POEMGA, ADA and Adagen^{®} were allowed to compete with Krystexxa^{®} for binding with anti-PEG antibodies in a positive plasma sample. **c**) and **d**) Direct and competitive assays described in panels a and b performed with a 55.6 kDa EG3 exendin-C-POEGMA conjugate. In all assays, the same unmodified peptide/protein content or similar PEG/OEG content in the case of polymer-modified samples per well were compared. See Methods section for details. Results are plotted as mean ± SEM, n=3 in panels a and b, n=5 in panels c and d. Data were analyzed by two-way ANOVA, followed by *post hoc* Dunnett's multiple comparison test (***P* < 0.01, and *****P* <0.0001).

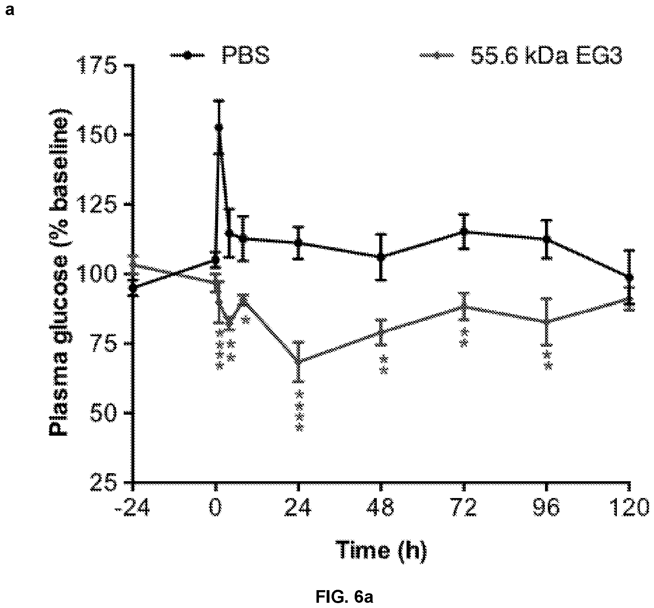

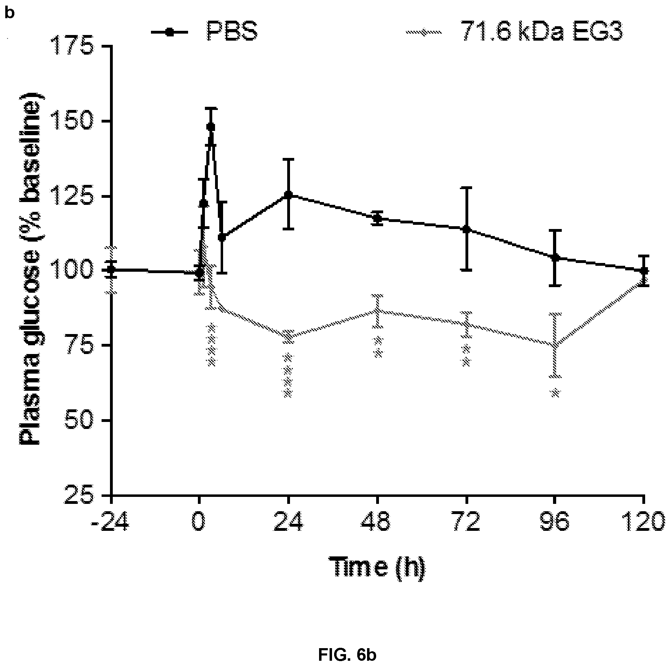

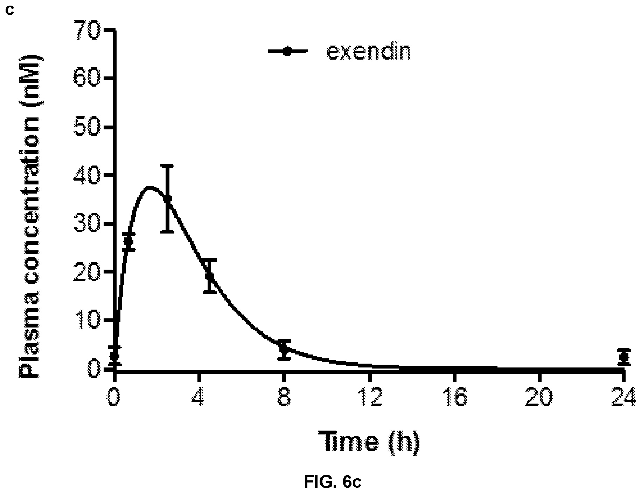

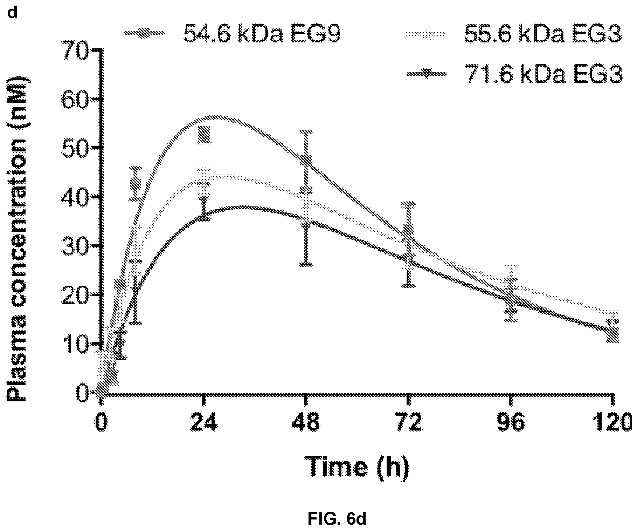

**FIG. 6**. Assessment of *in vivo* efficacy and pharmacokinetics of exendin-C-POEGMA conjugates. Blood glucose levels in fed mice measured before and after a single s.c. injection of **a**) 55.6 kDa and **b**) 71.6 kDa EG3 exendin-C-POEGMA conjugates at 25 nmol/kg or PBS at equivalent volume administered at t = 0 h. Blood glucose levels were normalized to the average glucose levels measured 24 h and immediately before injection. Data were analyzed by repeated measures two-way ANOVA, followed by *post hoc* Dunnett's multiple comparison test (n=5, **P* < 0.05, ***P <* 0.01, and *****P* <0.0001). **c**) Exendin and **d**) exendin-C-POEGMA conjugates (54.6 kDa EG9, 55.6 kDa EG3 and 71.6 kDa EG3) were fluorescently labeled with Alexa Fluor^{®} 488 and injected into mice (n=3) s.c. at 75 nmol/kg (45 nmol/kg fluorophore). Blood samples were collected via tail vein at various time points for fluorescence quantification. Data were analyzed using a non-compartmental fit (solid lines) to derive the pharmacokinetic parameters shown in **TABLE 2**. Results in all panels are plotted as mean ± SEM.

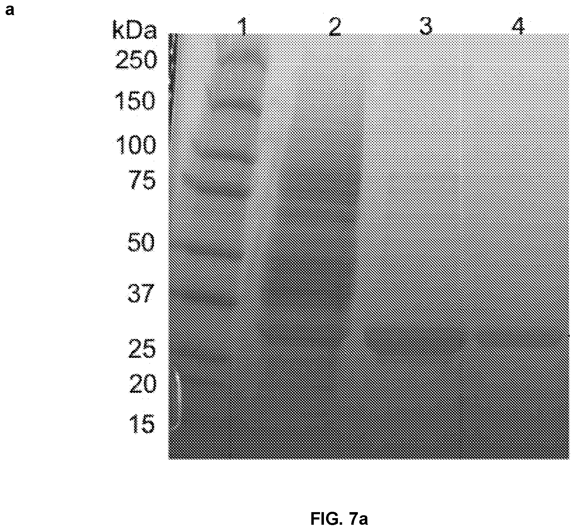

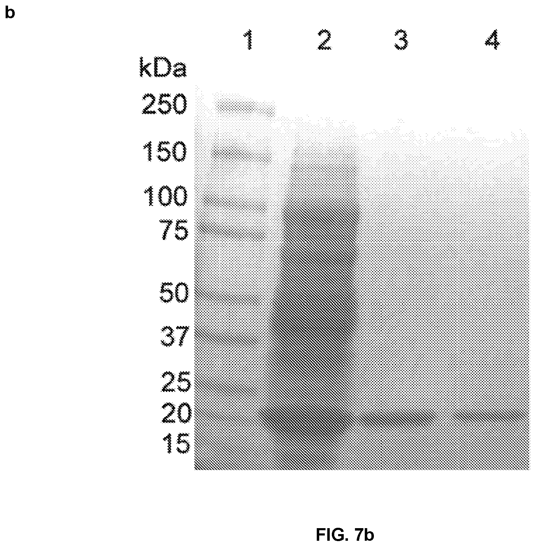

**FIG. 7****.** CuCl₂-stained SDS-PAGE analysis of **a**) exendin-srt-His₆-ELP purification by inverse transition cycling (ITC). Lane 1: marker, lane 2: E. *coli* lysate, lanes 3 and 4: soluble protein after one and two ITC cycles (yield: ~60 mg/L of culture). ELP: elastin-like polypeptide. **b**) His₆-sortase A purification by immobilized metal affinity chromatography (IMAC). Lane 1: marker, lane 2: *E. coli* lysate, lanes 3 and 4: first and second elution washes with imidazole (yield: ~400 mg/L of culture). His₆: hexahistidine.

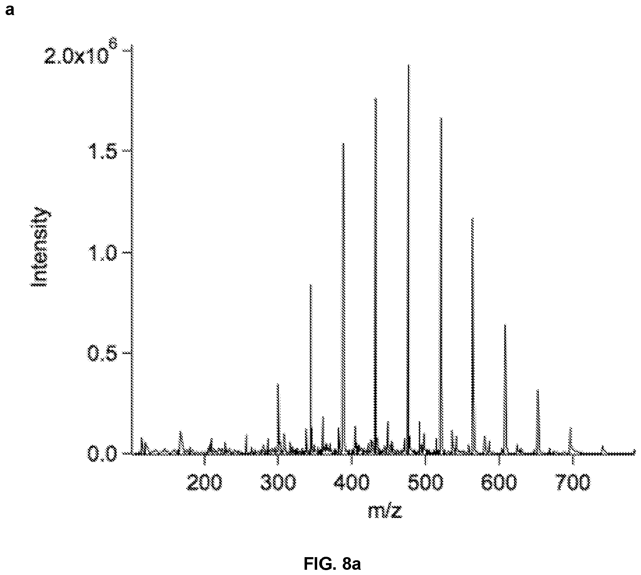

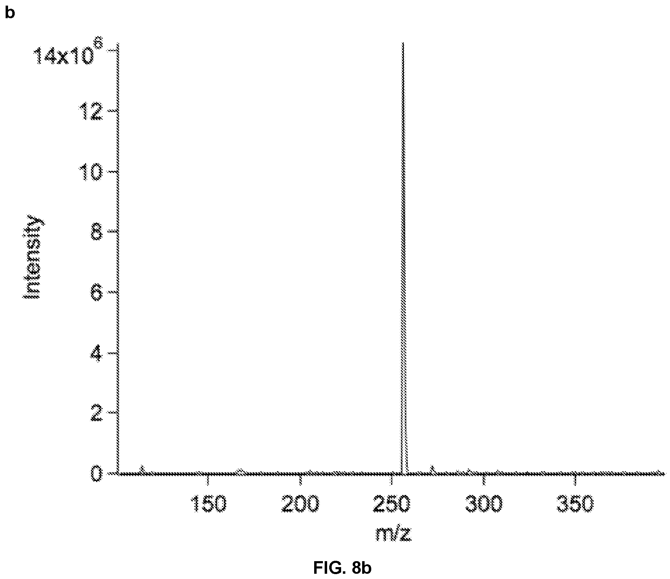

**FIG. 8****.** Liquid chromatography electrospray ionization mass spectrometry (LC/ESI-MS) characterization of OEGMA monomer with **a**) an average mass of ~ 500 Da or ~ 9 side-chain ethylene glycol repeats (EG9), and **b**) a mass of 232 Da or 3 side-chain EG repeats (EG3). Peaks were detected as [M+Na]⁺.

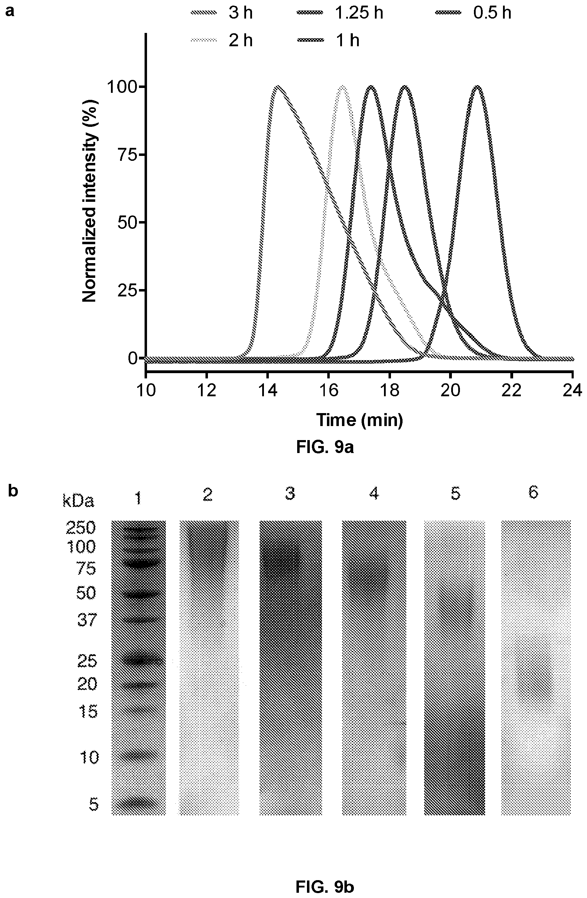

**FIG. 9****.** Physical characterization of EG9 exendin-C-POEGMA conjugates. a) SEC traces of ATRP reaction mixtures of grafting EG9 POEGMA from the exendin-C-Br macroinitiator carried out for various times with RI detection. Due to its small size and low concentration, the signal from the residual exendin-C-Br was too low to be observed by RI detection. **b**) Coomassie-stained SDS-PAGE analysis of EG9 exendin-C-POEGMA conjugates purified by a single round of preparative SEC. Lane 1: marker, from left to right in lanes 2-6: purified 155.0 kDa, 97.2 kDa, 66.2 kDa, 54.6 kDa and 25.4 kDa EG9 conjugates.

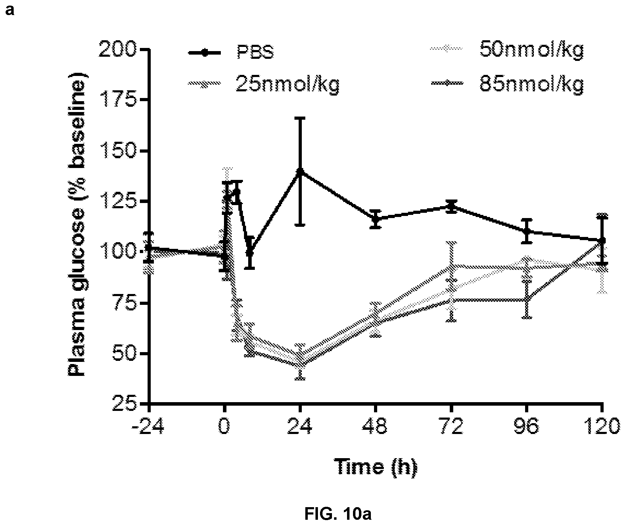

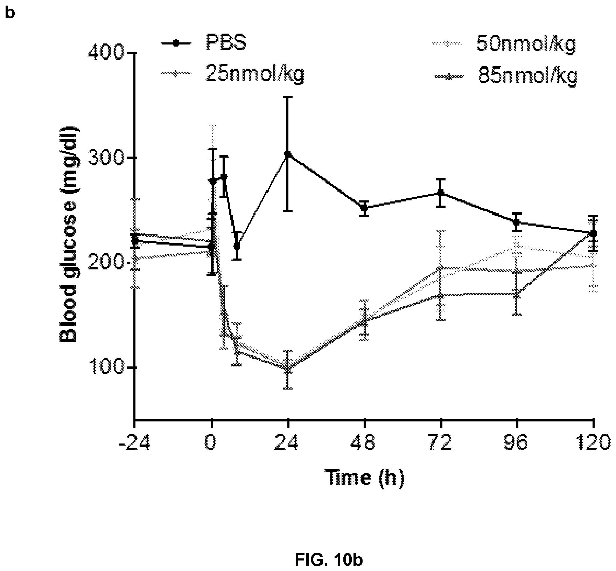

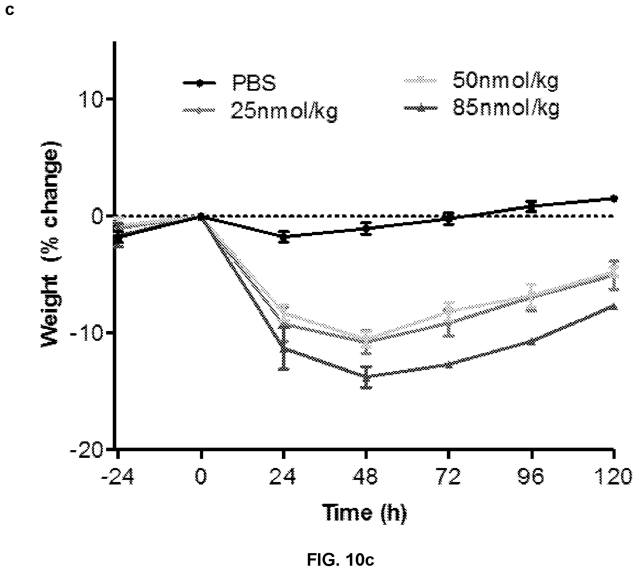

**FIG. 10****.** Assessment of *in vivo* dose-dependent efficacy of EG9 exendin-C-POEGMA. Overlaid **a**) normalized and **b**) un-normalized blood glucose levels of 6-wk-old male C57BL/6J mice (n=3) maintained on a 60 kCal% diet measured before and after a single s.c. injection of a 66.2 kDa EG9 exendin-C-POEGMA conjugate at 25, 50, 80 nmol/kg or phosphate buffered saline (PBS) control of equivalent volume administered at t = 0 h. Blood glucose levels in panel a were normalized to the average glucose levels measured 24 h prior to and immediately before injection. **c**) Overlaid weight profiles for all treatment and control groups. Weights are reported as % change from 0 h time point. Results in all panels are plotted as mean ± SEM.

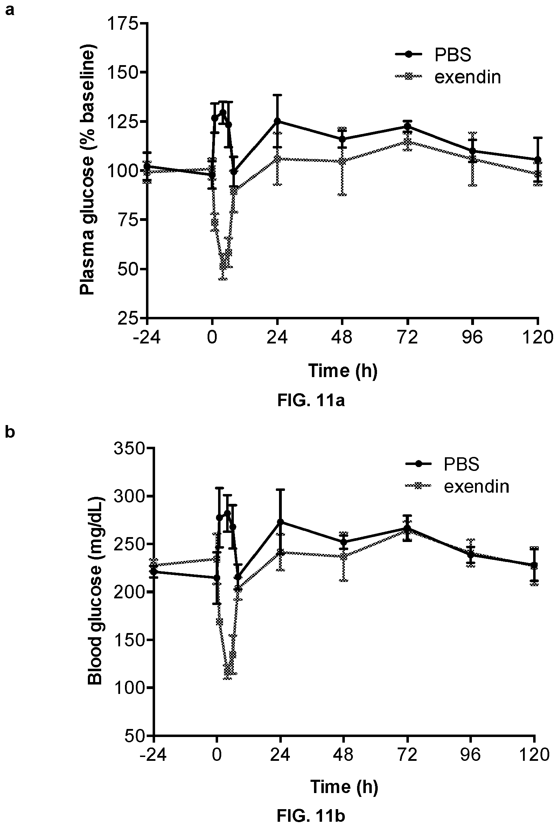

**FIG. 11** Assessment of *in vivo* efficacy of unmodified exendin. **a**) Normalized and **b**) un-normalized blood glucose profiles of fed mice (n=6) that received a single s.c. injection of unmodified exendin administered at 25 nmol/kg, compared to PBS control at equivalent volume injected at t = 0 h. Blood glucose levels in panel a were normalized to the average glucose levels measured 24 h prior to and immediately before injection. Results are plotted as mean ± SEM.

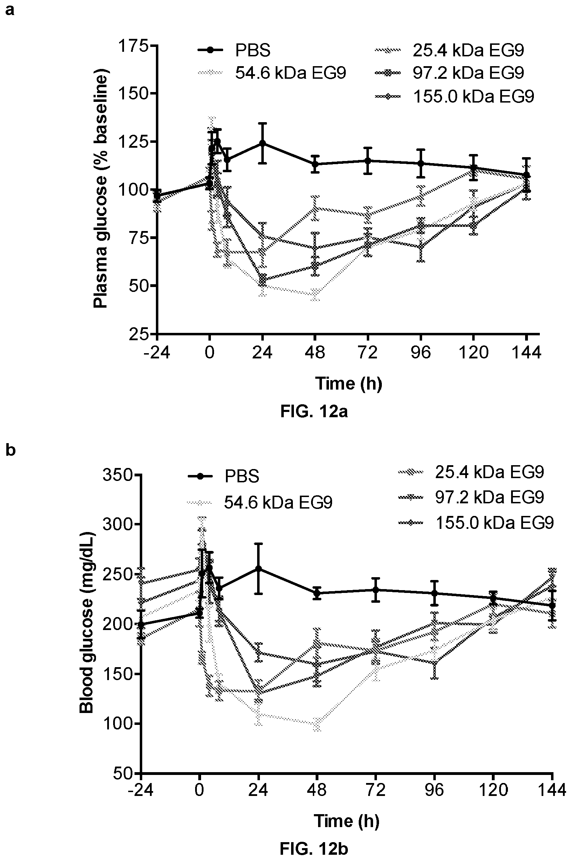

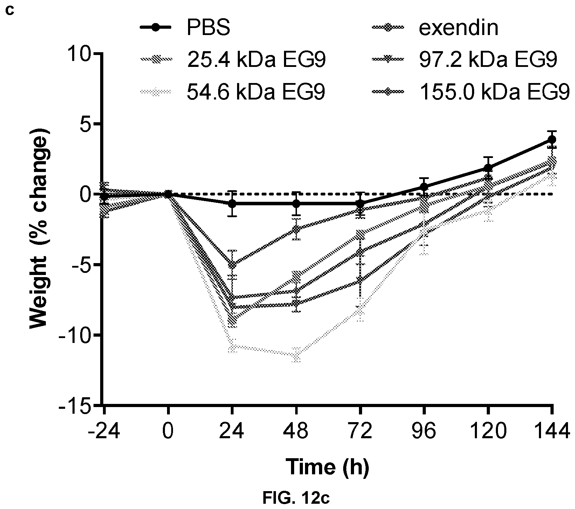

**FIG. 12****.** Assessment of *in vivo* MW-dependent efficacy of EG9 exendin-C-POEGMA conjugates. Overlaid **a**) normalized and **b**) un-normalized fed blood glucose levels in mice (n=6) measured before and after receiving a single s.c. injection of 25.4 kDa, 54.6 kDa, 97.2 kDa, 155.0 kDa EG9 exendin-POEGMA conjugates at 25 nmol/kg compared to PBS control at equivalent volume injected at t = 0 h. Blood glucose levels in panel a were normalized to the average glucose levels measured 24 h prior to and immediately before injection. **c**) Overlaid weight profiles for all treatment and control groups. Weights are reported as % change from 0 h time point. Weights were not measured for the exendin group at t = 144 h. Results in all panels are plotted as mean ± SEM.

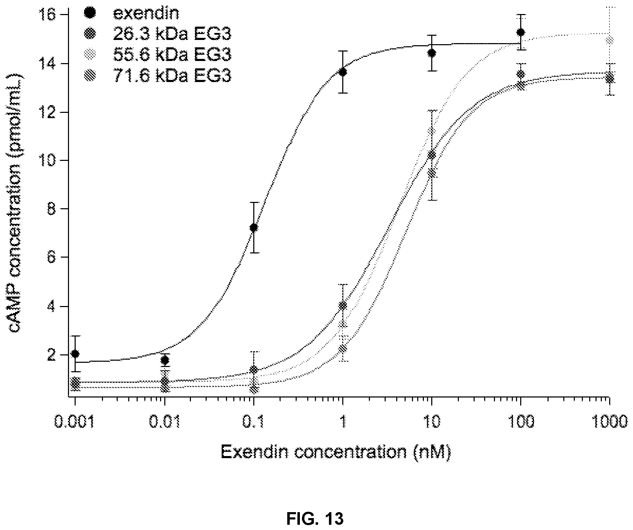

**FIG. 13****.** Cyclic adenosine monophosphate (cAMP) response of native exendin and EG3 exendin-C-POEGMA conjugates with Mₙs of 26.3 kDa, 55.6 kDa and 71.6 kDa in baby hamster kidney (BHK) cells expressing the glucagon-like peptide-1 receptor (GLP-1R). Results are plotted as mean ± SEM. Half-maximal effective concentration (EC₅₀) values are summarized in **TABLE 1.**

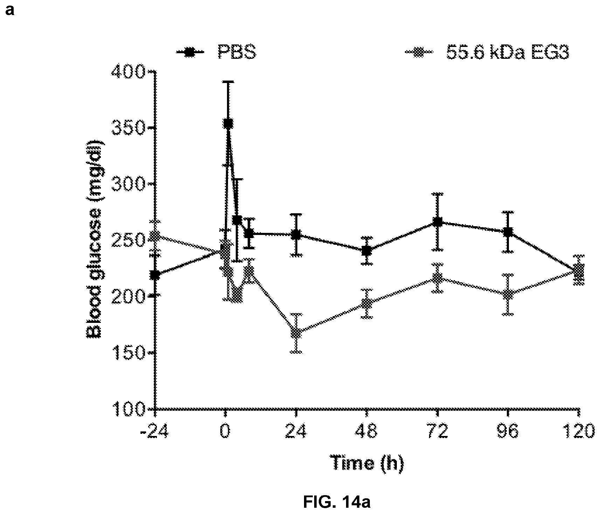

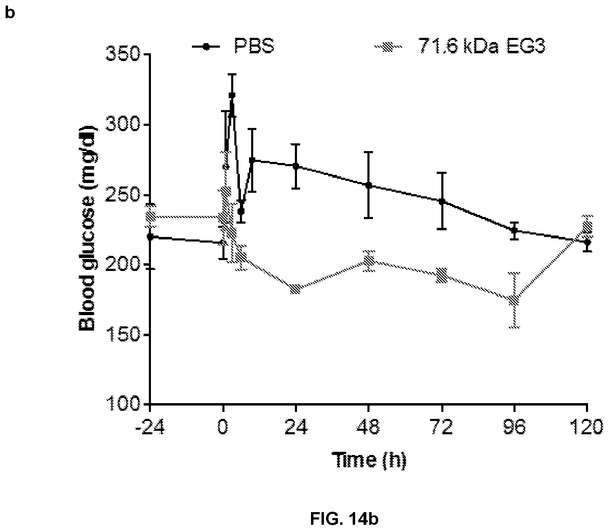

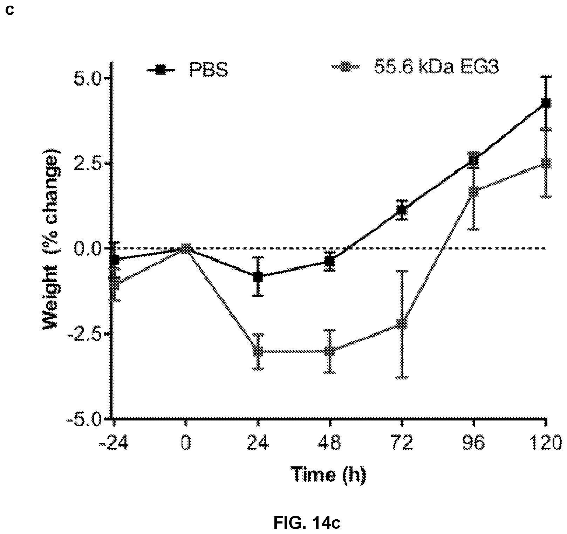

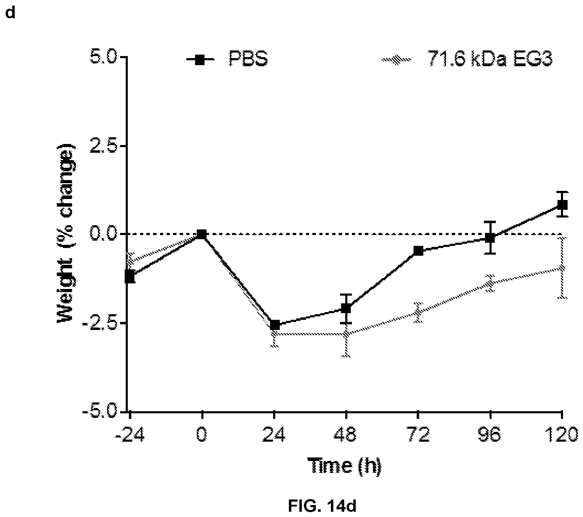

**FIG. 14****.** Assessment of *in vivo* efficacy of an EG3 exendin-C-POEGMA conjugate. Un-normalized blood glucose levels in fed mice (n=3) measured before and after receiving a single s.c. injection of **a**) 55.6 kDa and **b**) 71.6 kDa EG3 exendin-POEGMA conjugate at 25 nmol/kg compared to PBS control at equivalent volume administered at t = 0 h. Weight profiles for **c**) 55.6 kDa and **d**) 71.6 kDa EG3 exendin-C-POEGMA and PBS control groups. Weights are reported as % change from 0 h time point. Results in all panels are plotted as mean ± SEM.

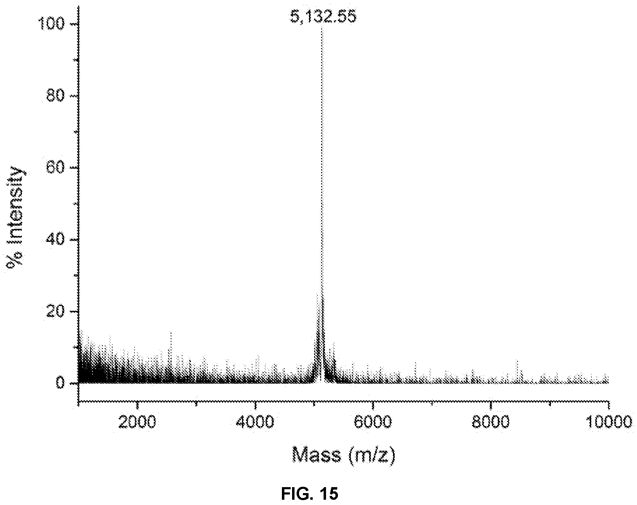

**FIG. 15****.** MALDI-MS spectrum of exendin-C-Br macroinitiator. Major peak at 5,132.55 Da agrees well with theoretical mass of 5,131.44 Da corresponding to a single AEBMP initiator attached to exendin.

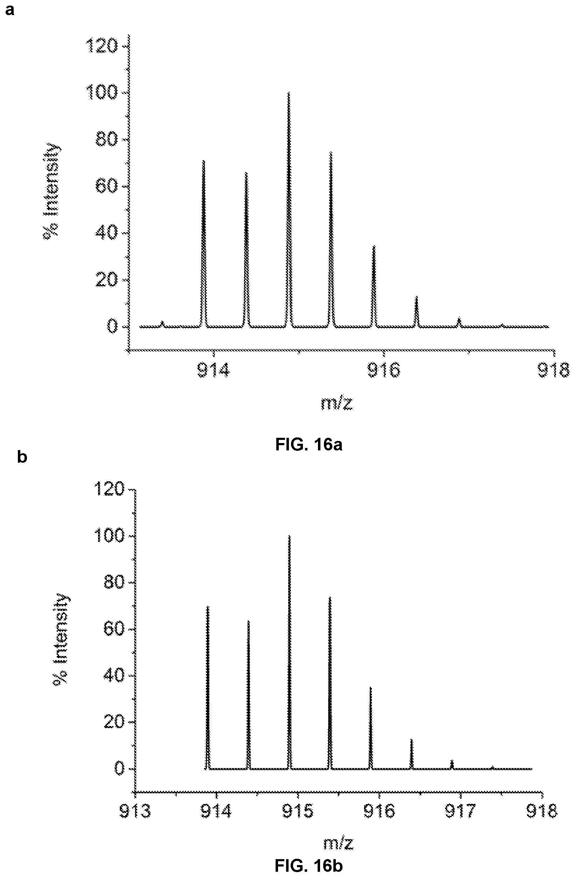

**FIG. 16****.** LC/MS-MS analysis of exendin-C-Br. **a**) Isotopic distribution of C-terminal peptide [NGGPSSGAPPPSLPET-"AEBMP", SEQ ID NO: 8]²⁺ detected by LC/MS-MS after trypsin digestion of exendin-C-Br. **b**) Theoretical isotopic distribution of C-terminal peptide of exendin-C-Br after trysin digestion generated by Molecular Mass Calculator software (Pacific Northwest National Laboratory).

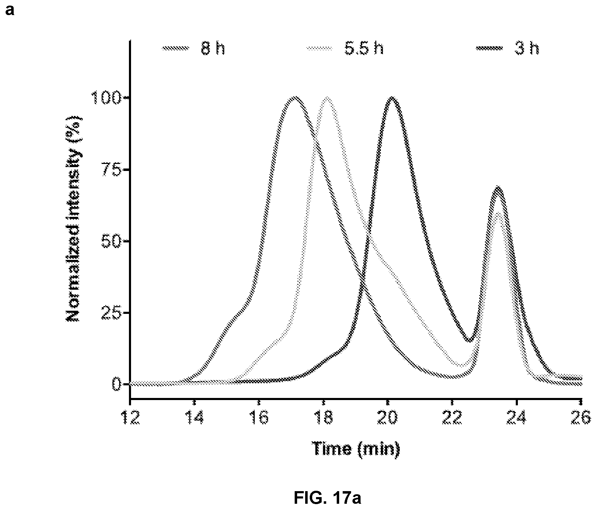

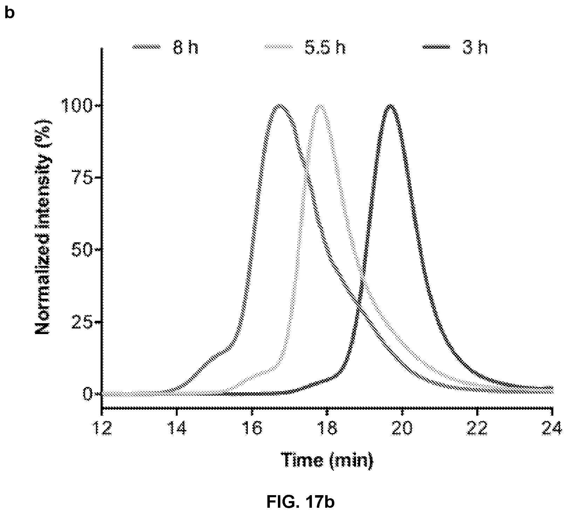

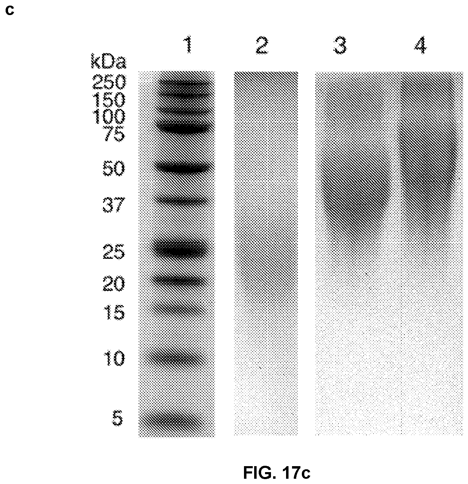

**FIG. 17****.** Physical characterization of EG3 exendin-C-POEGMA conjugates. SEC traces of EG3 exendin-C-POEGMA conjugates synthesized by *in situ* ATRP carried out for 2.5 h, 5.5 h, and 8 h, detected by **a**) UV-vis absorbance at 280 nm and **b**) RI. The signal from the residual exendin-C-Br was too low to be observed by RI detection due to its small size and low concentration. **c**) Coomassie-stained SDS-PAGE analysis of EG3 exendin-C-POEGMA conjugates purified by a single round of preparative SEC. Lane 1: marker, from left to right in lanes 2-4: purified 26.3, 55.6 and 71.6 kDa EG3 conjugates.

### DETAILED DESCRIPTION

Described herein are methods of reducing or eliminating the antigenicity of a molecule by conjugating a branched polymer thereto to form a molecule-polymer conjugate. The branched polymer may be conjugated to the molecule by a variety of ways. As detailed herein, sortase-catalyzed polymer conjugation may be used to generate a molecule-polymer conjugate. This strategy exploits the C-terminal native peptide ligation mechanism of the enzyme sortase A. Breaking up and appending PEG as short oligomeric side-chains of optimized length on the conjugated POEGMA not only retains the long circulation of the POEGMA conjugates, but also eliminates their reactivity toward patient-derived PEG antibodies. These results demonstrate that the architecture of PEG appended to a molecule plays a role in modulating its antigenicity. The compositions and methods detailed here may be used to deliver molecules with reduced or eliminated antigenicity, and thereby address the growing prevalence of pre-existing anti-PEG antibodies in the general population that is increasingly undermining the safety and efficacy of PEGylated therapeutics.

### 1. Definitions

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present invention. The materials, methods, and examples disclosed herein are illustrative only and not intended to be limiting.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "and" and "the" include plural references unless the context clearly dictates otherwise. The present disclosure also contemplates other embodiments "comprising," "consisting of" and "consisting essentially of," the embodiments or elements presented herein, whether explicitly set forth or not.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

The term "about" as used herein as applied to one or more values of interest, refers to a value that is similar to a stated reference value. In certain aspects, the term "about" refers to a range of values that fall within 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

"Amino acid" as used herein refers to naturally occurring and non-natural synthetic amino acids, as well as amino acid analogs and amino acid mimetics that function in a manner similar to the naturally occurring amino acids. Naturally occurring amino acids are those encoded by the genetic code. Amino acids can be referred to herein by either their commonly known three-letter symbols or by the one-letter symbols recommended by the IUPAC-IUB Biochemical Nomenclature Commission. Amino acids include the side chain and polypeptide backbone portions.

"Antigen" refers to a molecule capable of being bound by an antibody or a T cell receptor. The term "antigen", as used herein, also encompasses T-cell epitopes. An antigen is additionally capable of being recognized by the immune system and/or being capable of inducing a humoral immune response and/or cellular immune response leading to the activation of B-lymphocytes and/or T-lymphocytes. In some embodiments, the antigen contains or is linked to a Th cell epitope. An antigen can have one or more epitopes (B-epitopes and T-epitopes). Antigens may include polypeptides, polynucleotides, carbohydrates, lipids, small molecules, and combinations thereof. Antigens may also be mixtures of several individual antigens. "Antigenicity" refers to the ability of an antigen to specifically bind to a T cell receptor or antibody and includes the reactivity of an antigen toward pre-existing antibodies in a subject. "Immunogenicity" refers to the ability of any antigen to induce an immune response and includes the intrinsic ability of an antigen to generate antibodies in a subject. As used herein, the terms "antigenicity" and "immunogenicity" are different and not interchangeable.

The terms "control," "reference level," and "reference" are used herein interchangeably. The reference level may be a predetermined value or range, which is employed as a benchmark against which to assess the measured result. "Control group" as used herein refers to a group of control subjects. The predetermined level may be a cutoff value from a control group. The predetermined level may be an average from a control group. Cutoff values (or predetermined cutoff values) may be determined by Adaptive Index Model (AIM) methodology. Cutoff values (or predetermined cutoff values) may be determined by a receiver operating curve (ROC) analysis from biological samples of the patient group. ROC analysis, as generally known in the biological arts, is a determination of the ability of a test to discriminate one condition from another, e.g., to determine the performance of each marker in identifying a patient having CRC. A description of ROC analysis is provided in P.J. Heagerty et al. (Biometrics 2000, 56, 337-44), the disclosure of which is hereby incorporated by reference in its entirety. Alternatively, cutoff values may be determined by a quartile analysis of biological samples of a patient group. For example, a cutoff value may be determined by selecting a value that corresponds to any value in the 25th-75th percentile range, preferably a value that corresponds to the 25th percentile, the 50th percentile or the 75th percentile, and more preferably the 75th percentile. Such statistical analyses may be performed using any method known in the art and can be implemented through any number of commercially available software packages (e.g., from Analyse-it Software Ltd., Leeds, UK; StataCorp LP, College Station, TX; SAS Institute Inc., Cary, NC.). The healthy or normal levels or ranges for a target or for a protein activity may be defined in accordance with standard practice. A control may be a molecule, or sample comprising a molecule, without having a branched polymer conjugated thereto. A control may be a molecule, or sample comprising a molecule, with a polymer, that is different from a branched polymer as detailed herein, conjugated thereto. A control may be a subject, or a sample therefrom, whose disease state is known. The subject, or sample therefrom, may be healthy, diseased, diseased prior to treatment, diseased during treatment, or diseased after treatment, or a combination thereof. The control may include, for example, the molecule alone or by itself, the molecule conjugated to a different polymer, the molecule conjugated to a non-branched polymer or to a polymer that is not branched, the molecule conjugated to PEG, the molecule conjugated to unbranched PEG, the molecule directly conjugated to a linear polymer, or the molecule conjugated to a side chain directly (without a branched polymer).

The term "expression vector" indicates a plasmid, a virus or another medium, known in the art, into which a nucleic acid sequence for encoding a desired protein can be inserted or introduced.

The term "host cell" is a cell that is susceptible to transformation, transfection, transduction, conjugation, and the like with a nucleic acid construct or expression vector. Host cells can be derived from plants, bacteria, yeast, fungi, insects, animals, etc. In some embodiments, the host cell includes *Escherichia coli.*

"Opsonization" refers to the molecular mechanism whereby molecules, microbes, or apoptotic cells are chemically modified to have stronger interactions with cell surface receptors on phagocytes and natural killer (NK) cells. An antigen on the molecules, microbes, or apoptotic cell is coated in opsonins. The opsonins enhance binding to immune cells such as macrophages and neutrophils. Opsonization also mediates phagocytosis via signal cascades from cell surface receptors.

"Polymer" or "synthetic polymer" refers to a polymer which is produced from at least one monomer by a chemical process. A synthetic polymer is not produced directly by a living organism. Synthetic polymers include a homopolymer, heteropolymer, block polymer, copolymer, ter-polymer, etc., and blends, combinations and mixtures thereof. Examples of synthetic polymers include, but are not limited to, functionalized polymers, such as a polymer comprising 5-vinyltetrazole monomer units and having a molecular weight distribution less than 2.0. A synthetic polymer may be or contain one or more of a star block copolymer, a linear polymer, a branched polymer, a hyperbranched polymer, a dendritic polymer, a comb polymer, a graft polymer, a brush polymer, a bottle-brush copolymer and a crosslinked structure, such as a block copolymer comprising a block of 5-vinyltetrazole monomer units. Synthetic polymers include, without limitation, polyesters, poly(meth)acrylamides, poly(meth)acrylates, polyethers, polystyrenes, polynorbornenes and monomers that have unsaturated bonds. For example, amphiphilic comb polymers are described in U.S. Patent Application Publication No. 2007/0087114 and in U.S. Patent No. 6,207,749 to Mayes et al. The amphiphilic comb-type polymers may be present in the form of copolymers, containing a backbone formed of a hydrophobic, water-insoluble polymer and side chains formed of short, hydrophilic non-cell binding polymers. Examples of other synthetic polymers include, but are not limited to, polyalkylenes such as polyethylene and polypropylene and polyethyleneglycol (PEG); polychloroprene; polyvinyl ethers; such as poly(vinyl acetate); polyvinyl halides such as poly(vinyl chloride); polysiloxanes; polystyrenes; polyurethanes; polyacrylates; such as poly(methyl (meth)acrylate), poly(ethyl (meth)acrylate), poly(n-butyl(meth)acrylate), poly(isobutyl (meth)acrylate), poly(tert-butyl (meth)acrylate), poly(hexyl(meth)acrylate), poly(isodecyl (meth)acrylate), poly(lauryl (meth)acrylate), poly(phenyl (meth)acrylate), poly(methyl acrylate), poly(isopropyl acrylate), poly(isobutyl acrylate), and poly(octadecyl acrylate); polyacrylamides such as poly(acrylamide), poly(methacrylamide), poly(ethyl acrylamide), poly(ethyl methacrylamide), poly(N-isopropyl acrylamide), poly(n, iso, and tert-butyl acrylamide); and copolymers and mixtures thereof. These synthetic polymers may include useful derivatives, including synthetic polymers having substitutions, additions of chemical groups, for example, alkyl groups, alkylene groups, hydroxylations, oxidations, and other modifications routinely made by those skilled in the art. The synthetic polymers may include zwitterionic polymers such as, for example, polyphosphorycholine, polycarboxybetaine, and polysulfobetaine. The synthetic polymers may have side chains of betaine, carboxybetaine, sulfobetaine, oligoethylene glycol (OEG), sarcosine or polyethyleneglycol (PEG).

"Polynucleotide" as used herein can be single stranded or double stranded, or can contain portions of both double stranded and single stranded sequence. The polynucleotide can be nucleic acid, natural or synthetic, DNA, genomic DNA, cDNA, RNA, or a hybrid, where the polynucleotide can contain combinations of deoxyribo- and ribo-nucleotides, and combinations of bases including uracil, adenine, thymine, cytosine, guanine, inosine, xanthine hypoxanthine, isocytosine, and isoguanine. Polynucleotides can be obtained by chemical synthesis methods or by recombinant methods.

A "peptide" or "polypeptide" is a linked sequence of two or more amino acids linked by peptide bonds. The polypeptide can be natural, synthetic, or a modification or combination of natural and synthetic. Peptides and polypeptides include proteins such as binding proteins, receptors, and antibodies. The terms "polypeptide", "protein," and "peptide" are used interchangeably herein. "Primary structure" refers to the amino acid sequence of a particular peptide. "Secondary structure" refers to locally ordered, three dimensional structures within a polypeptide. These structures are commonly known as domains, e.g., enzymatic domains, extracellular domains, transmembrane domains, pore domains, and cytoplasmic tail domains. Domains are portions of a polypeptide that form a compact unit of the polypeptide and are typically 15 to 350 amino acids long. Exemplary domains include domains with enzymatic activity or ligand binding activity. Typical domains are made up of sections of lesser organization such as stretches of beta-sheet and alpha-helices. "Tertiary structure" refers to the complete three dimensional structure of a polypeptide monomer. "Quaternary structure" refers to the three dimensional structure formed by the noncovalent association of independent tertiary units. A "motif" is a portion of a polypeptide sequence and includes at least two amino acids. A motif may be 2 to 20, 2 to 15, or 2 to 10 amino acids in length. In some embodiments, a motif includes 3, 4, 5, 6, or 7 sequential amino acids.

"Pharmacokinetics" as used herein refers the circulation of a drug or molecule in the body and its bioavailability, distribution, and excretion.

"Recombinant" when used with reference, e.g., to a cell, or nucleic acid, protein, or vector, indicates that the cell, nucleic acid, protein, or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed, or not expressed at all

"Sample" or "test sample" as used herein can mean any sample in which the presence and/or level of a target is to be detected or determined or any sample comprising a molecule or conjugate as described herein. Samples may include liquids, solutions, emulsions, or suspensions. Samples may include a medical sample. Samples may include any biological fluid or tissue, such as blood, whole blood, fractions of blood such as plasma and serum, muscle, interstitial fluid, sweat, saliva, urine, tears, synovial fluid, bone marrow, cerebrospinal fluid, nasal secretions, sputum, amniotic fluid, bronchoalveolar lavage fluid, gastric lavage, emesis, fecal matter, lung tissue, peripheral blood mononuclear cells, total white blood cells, lymph node cells, spleen cells, tonsil cells, cancer cells, tumor cells, bile, digestive fluid, skin, or combinations thereof. In some embodiments, the sample comprises an aliquot. In other embodiments, the sample comprises a biological fluid. Samples can be obtained by any means known in the art. The sample can be used directly as obtained from a patient or can be pretreated, such as by filtration, distillation, extraction, concentration, centrifugation, inactivation of interfering components, addition of reagents, and the like, to modify the character of the sample in some manner as discussed herein or otherwise as is known in the art.

"Sortase" refers to a polypeptide that recognizes a sortase recognition site in a protein and cleaves a peptide bond therein, forming a stable intermediate that joins the catalytic thiol of sortase to the carboxyl group of an amino acid within the recognition site via a thioester bond. This intermediate undergoes nucleophilic attack by the α-amino group of an oligoglycine branch in the peptidoglycan, generating a native peptide bond that anchors the substrate protein to the cell wall. Sortase A (SrtA) may recognize a sortase A recognition site, such as an amino acid sequence consisting of LPXZG (SEQ ID NO: 3, where X and Z are independently any amino acid) and cleave the peptide bond between the Z amino acid and the glycine of LPXZG and form a thioester bond between the catalytic thiol in SrtA and the carboxyl group of the Z amino acid. The thioester bond between the catalytic thiol in SrtA and the carboxyl group of the Z amino acid forms an intermediate, and the intermediate undergoes nucleophilic attack by the ε-amino group of the lysine of first polypeptide to form an isopeptide bond between the ε-amino group of the lysine and the Z amino acid of LPXZG. In some embodiments, SrtA forms an isopeptide bond between the ε-amino group of any solvent-accessible, nucleophilic lysine of the first polypeptide and the Z amino acid of LPXZG. In some embodiments, the sortase A recognition site includes LPXTG (SEQ ID NO: 1, where X is any amino acid). The SrtA may be any SrtA, such as *Staphylococcus aureus* SrtA. SrtA may be from a Gram positive bacterium, such as, for example, bacteria in a genus selected from *Staphylococcus, Streptococcus, Enterococcus, Bacillus, Corynebacterium, Nocardia, Clostridium, Actinobacteria,* and *Listeria.* In some embodiments, SrtA is from S. *aureaus.* The SrtA may be wild-type SrtA or a variant thereof. Sortase is further detailed in International Patent Application No. PCT/US2015/017601, filed February 25, 2015, published as WO 2015/130846, and International Patent Application No. PCT/US2014/040319, filed May 30, 2014, published as WO 2014/194244.

"Stealth" or "stealth polymer" refers to a molecule-polymer conjugate, or to the polymer thereof, that can remain undetected by immune cells in the bloodstream for a prolonged period of time. Stealth molecule-polymer conjugates are at least partially resistant to enzymatic degradation of the conjugate, or to the polypeptide thereof, such as by proteases, and opsonization, which is a common method used by immune system to recognize foreign particles. Accordingly, stealth molecule-polymer conjugates may have one or more of reduced antigenicity, reduced immunogenicity, increased stability, increased half-life, and increased bioavailability relative to other polymers, conjugates, non-stealth polymers, and/or non-stealth conjugates. The ability to delay, reduce, or prevent opsonization, recognition by the immune system, or clearance of a conjugate (or the polypeptide or molecules thereof) from the body may be referred to herein as a stealth property.

"Subject" as used herein can mean a mammal that wants or is in need of the herein described conjugates. The subject may be a patient. The subject may be a human or a non-human animal. The subject may be a mammal. The mammal may be a primate or a non-primate. The mammal can be a primate such as a human; a non-primate such as, for example, dog, cat, horse, cow, pig, mouse, rat, camel, llama, goat, rabbit, sheep, hamster, and guinea pig; or non-human primate such as, for example, monkey, chimpanzee, gorilla, orangutan, and gibbon. The subject may be of any age or stage of development, such as, for example, an adult, an adolescent, or an infant.

"Target" as used herein can refer to an entity that a molecule binds. A target may include, for example, a small molecule, a protein, a polypeptide, a polynucleotide, a carbohydrate, or a combination thereof.

"Treatment" or "treating," when referring to protection of a subject from a disease, means preventing, suppressing, repressing, ameliorating, or completely eliminating the disease. Preventing the disease involves administering a composition of the present invention to a subject prior to onset of the disease. Suppressing the disease involves administering a composition of the present invention to a subject after induction of the disease but before itsclinical appearance. Repressing or ameliorating the disease involves administering a composition of the present invention to a subject after clinical appearance of the disease.

"Variant" as used herein with respect to a polynucleotide means (i) a portion or fragment of a referenced nucleotide sequence; (ii) the complement of a referenced nucleotide sequence or portion thereof; (iii) a polynucleotide that is substantially identical to a referenced polynucleotide or the complement thereof; or (iv) a polynucleotide that hybridizes under stringent conditions to the referenced polynucleotide, complement thereof, or a sequences substantially identical thereto.

A "variant" can further be defined as a peptide or polypeptide that differs in amino acid sequence by the insertion, deletion, or conservative substitution of amino acids, but retain at least one biological activity. Representative examples of "biological activity" include the ability to be bound by a specific antibody or polypeptide or to promote an immune response. Variant can mean a substantially identical sequence. Variant can mean a functional fragment thereof. Variant can also mean multiple copies of a polypeptide. The multiple copies can be in tandem or separated by a linker. Variant can also mean a polypeptide with an amino acid sequence that is substantially identical to a referenced polypeptide with an amino acid sequence that retains at least one biological activity. A conservative substitution of an amino acid, i.e., replacing an amino acid with a different amino acid of similar properties (e.g., hydrophilicity, degree and distribution of charged regions) is recognized in the art as typically involving a minor change. These minor changes can be identified, in part, by considering the hydropathic index of amino acids. See Kyte et al., J. Mol. Biol. 1982, 157, 105-132. The hydropathic index of an amino acid is based on a consideration of its hydrophobicity and charge. It is known in the art that amino acids of similar hydropathic indexes can be substituted and still retain protein function. In one aspect, amino acids having hydropathic indices of ±2 are substituted. The hydrophobicity of amino acids can also be used to reveal substitutions that would result in polypeptides retaining biological function. A consideration of the hydrophilicity of amino acids in the context of a polypeptide permits calculation of the greatest local average hydrophilicity of that polypeptide, a useful measure that has been reported to correlate well with antigenicity and immunogenicity, as discussed in U.S. Patent No. 4,554,101. Substitution of amino acids having similar hydrophilicity values can result in polypeptides retaining biological activity, for example immunogenicity, as is understood in the art. Substitutions can be performed with amino acids having hydrophilicity values within ±2 of each other. Both the hydrophobicity index and the hydrophilicity value of amino acids are influenced by the particular side chain of that amino acid. Consistent with that observation, amino acid substitutions that are compatible with biological function are understood to depend on the relative similarity of the amino acids, and particularly the side chains of those amino acids, as revealed by the hydrophobicity, hydrophilicity, charge, size, and other properties.

A variant can be a polynucleotide sequence that is substantially identical over the full length of the full gene sequence or a fragment thereof. The polynucleotide sequence can be 80%, 81%, 82%, 83%, 84%, 85%, 86%, 87%, 88%, 89%, 90%, 91%, 92%, 93%, 94%, 95%, 96%, 97%, 98%, 99%, or 100% identical over the full length of the gene sequence or a fragment thereof. A variant can be an amino acid sequence that is substantially identical over the full length of the amino acid sequence or fragment thereof. The amino acid sequence can be 80%, 81%, 82%, 83%, 84%, 85%, 86%, 87%, 88%, 89%, 90%, 91%, 92%, 93%, 94%, 95%, 96%, 97%, 98%, 99%, or 100% identical over the full length of the amino acid sequence or a fragment thereof.

The term "acyl" or "carbonyl" refers to the group -C(O)R wherein R is selected from the group consisting of hydrogen, alkyl, alkenyl, alkynyl, aryl, cycloalkyl, heterocyclyl, heteroaryl, arylalkyl, cycloalkylalkyl, heteroarylalkyl and heterocyclylalkyl, any of which may be optionally substituted, e.g., with one or more substituents. For example, when R is alkyl, such a group may be referred to as an alkylcarbonyl group.

The term "alkoxy" refers to the group -O-R wherein R is alkyl, alkenyl, alkynyl, cycloalkyl or heterocyclyl, any of which may be optionally substituted, e.g., with one or more substituents.

The term "alkyl" refers to a straight or branched hydrocarbon chain, containing the indicated number of carbon atoms. For example, C₁-C₁₂ alkyl indicates that the alkyl group may have from 1 to 12 (inclusive) carbon atoms, and C₁-C₄ alkyl indicates that the alkyl group may have from 1 to 4 (inclusive) carbon atoms. An alkyl group may be optionally substituted. Examples of C₁-C₄ alkyl groups include methyl, ethyl, n-propyl, isopropyl, n-butyl, sec-butyl and tert-butyl.

The term "alkenyl" refers to a straight or branched hydrocarbon chain having one or more double bonds. Examples of alkenyl groups include, but are not limited to, allyl, propenyl, 2-butenyl, 3-hexenyl and 3-octenyl groups. One of the double bond carbons may optionally be the point of attachment of the alkenyl substituent. An alkenyl group may be optionally substituted.

The term "alkynyl" refers to a straight or branched hydrocarbon chain having one or more triple bonds. Examples of alkynyl groups include, but are not limited to, ethynyl, propargyl, and 3-hexynyl. One of the triple bond carbons may optionally be the point of attachment of the alkynyl substituent. An alkynyl group may be optionally substituted.

The term "aryl" refers to an aromatic monocyclic, bicyclic, or tricyclic hydrocarbon ring system, wherein any ring atom capable of substitution can be substituted (e.g., with one or more substituents). Examples of aryl moieties include, but are not limited to, phenyl, naphthyl, and anthracenyl. An aromatic amine is an aryl group substituted with one or more amino groups. An aromatic alcohol is an aryl group substituted with one or more hydroxyl groups. Both aromatic amines and aromatic alcohols may be further substituted with other substitutents.

The term "arylalkyl" refers to an alkyl moiety in which an alkyl hydrogen atom is replaced with an aryl group. Arylalkyl includes groups in which more than one hydrogen atom has been replaced with an aryl group. Examples of arylalkyl groups include benzyl, 2-phenylethyl, 3-phenylpropyl, 9-fluorenyl, benzhydryl, and trityl groups.

The term "carboxyl" refers to the group -C(=O)OR, wherein R is selected from the group consisting of hydrogen, alkyl, alkenyl, alkynyl, aryl, cycloalkyl, heterocyclyl, heteroaryl, arylalkyl, cycloalkylalkyl, heteroarylalkyl and heterocyclylalkyl any of which may be optionally substituted, e.g., with one or more substituents.

The term "carboxylate" refers to the group ―C(=O)O⁽⁻⁾.

The term "carbonylamino" or "amido" refers to the group ―C(O)NR'R" wherein R' and R" are independently selected from the group consisting of hydrogen, alkyl, alkenyl, alkynyl, aryl, cycloalkyl, heterocyclyl, heteroaryl, arylalkyl, cycloalkylalkyl, heteroarylalkyl, and heterocyclylalkyl, or R' and R" together with the nitrogen to which they are attached, may form a ring. The groups R' and R" may be optionally substituted, e.g., with one or more substituents, or when R' and R" together with the nitrogen to which they are attached form a ring, the ring may be optionally substituted, e.g., with one or more substituents.

The term "amide" refers to the group -C(O)NR wherein R is selected from the group consisting of hydrogen, alkyl, alkenyl, alkynyl, aryl, cycloalkyl, heterocyclyl, heteroaryl, arylalkyl,cycloalkylalkyl, heteroarylalkyl and heterocyclylalkyl, any of which may be optionally substituted, e.g., with one or more substituents.

The term "amine" refers to the group ―NH₂.

The term "cycloalkyl" as used herein refers to nonaromatic, saturated or partially unsaturated cyclic, bicyclic, tricyclic or polycyclic hydrocarbon groups having 3 to 12 carbons (e.g., 3, 4, 5, 6, or 7 carbon atoms). Any ring atom can be substituted (e.g., with one or more substituents). Cycloalkyl groups can contain fused rings. Fused rings are rings that share one or more common carbon atoms. Examples of cycloalkyl groups include, but are not limited to, cyclopropyl, cyclobutyl, cyclopentyl, cyclohexyl, cyclohexenyl, cyclohexadienyl, methylcyclohexyl, adamantyl, norbornyl and norbornenyl.

The term "ester" refers to the group -C(O)OR wherein R is selected from the group consisting of hydrogen, alkyl, alkenyl, alkynyl, aryl, cycloalkyl, heterocyclyl, heteroaryl, arylalkyl, cycloalkylalkyl, heteroarylalkyl and heterocyclylalkyl, any of which may be optionally substituted, e.g., with one or more substituents.

The term "halo" or "halogen" as used herein refers to any radical of fluorine, chlorine, bromine or iodine.

The term "heteroaryl" as used herein refers to an aromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms independently selected from O, N, S, P and Si (e.g., carbon atoms and 1-3, 1-6, or 1-9 heteroatoms independently selected from O, N, S, P and Si if monocyclic, bicyclic, or tricyclic, respectively). Any ring atom can be substituted (e.g., with one or more substituents). Heteroaryl groups can contain fused rings, which are rings that share one or more common atoms. Examples of heteroaryl groups include, but are not limited to, radicals of pyridine, pyrimidine, pyrazine, pyridazine, pyrrole, imidazole, pyrazole, oxazole, isoxazole, furan, thiazole, isothiazole, thiophene, quinoline, isoquinoline, quinoxaline, quinazoline, cinnoline, indole, isoindole, indolizine, indazole, benzimidazole, phthalazine, pteridine, carbazole, carboline, phenanthridine, acridine, phenanthroline, phenazine, naphthyridines and purines.

The term "heterocyclyl" as used herein refers to a nonaromatic, saturated or partially unsaturated 3-10 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, S, Si and P (e.g., carbon atoms and 1-3, 1-6, or 1-9 heteroatoms of O, N, S, Si and P if monocyclic, bicyclic, or tricyclic, respectively). Any ring atom can be substituted (e.g., with one or more substituents). Heterocyclyl groups can contain fused rings, which are rings that share one or more common atoms. Examples of heterocyclyl groups include, but are not limited to, radicals of tetrahydrofuran, tetrahydrothiophene, tetrahydropyran, piperidine, piperazine, morpholine, pyrroline, pyrimidine, pyrrolidine, indoline, tetrahydropyridine, dihydropyran, thianthrene, pyran, benzopyran, xanthene, phenoxathiin, phenothiazine, furazan, lactones, lactams such as azetidinones and pyrrolidinones, sultams, sultones, and the like.

The term "hydroxy" refers to an -OH radical. The term "alkoxy" refers to an -O-alkyl radical. The term "aryloxy" refers to an ―O-aryl radical. The term "haloalkoxy" refers to an -O-haloalkyl radical.

Where chemical groups are specified by their conventional chemical formulae, written from left to right, they optionally encompass substituents resulting from writing the structure from right to left, e.g., -CH₂O- optionally also recites -OCH₂-.

### 2. Conjugate

Provided herein is a molecule-polymer conjugate. The molecule-polymer conjugate includes a branched polymer and a molecule covalently attached thereto. The molecule-polymer may include more than one branched polymer conjugated to the molecule. In some embodiments, more than one branched polymer is conjugated to the molecule, each branched polymer conjugated to a different site of the molecule.

### a. Branched Polymer

The branched polymer comprises poly[oligo(ethylene glycol) methyl ether methacrylate] (POEGMA), wherein the POEGMA comrprises a backbone and a plurality of side chains. Each side chain is covalently attached to the backbone.

### i) Backbone

The backbone comprises poly(methyl methacrylate). The molecule may be conjugated to the backbone of the branched polymer.

### ii) Side Chains

According to the present invention, the side chains are polymers, each side chain covalently attached to the backbone. Each side chain comprises 2 to 7 monomers of ethylene glycol (EG) repeated in tandem.

According to the invention presently claimed, each side chain includes 2 to 7 monomers, 3 to 7 monomers, 4 to 7 monomers, 5 to 7 monomers,, or 3 to 5 monomers repeated in tandem. Each side chain may include at least 3 monomers, at least 4 monomers, at least 5 monomers, or at least 6 monomers repeated in tandem. Each side chain may include less than 7 monomers, less than 6 monomers, less than 5 monomers, less than 4 monomers, or less than 3 monomers repeated in tandem. In some embodiments, each side chain comprises at least 2 monomers repeated in tandem. In some embodiments, each side chain comprises at least 3 monomers repeated in tandem. In some embodiments, each side chain comprises 3 monomers repeated in tandem.

The monomer of each side chain comprises ethylene glycol. In some embodiments, each side chain includes at least 2 monomers of ethylene glycol (EG) repeated in tandem. In some embodiments, each side chain comprises 3 monomers of ethylene glycol (EG) repeated in tandem. In some embodiments, each side chain comprises 3 to 7 monomers of ethylene glycol (EG) repeated in tandem. Adjacent side chains may be the same. Adjacent side chains may be different from each other.

Each side chain has a first terminal end and a second terminal end. The first terminal end is covalently attached to the backbone. The second terminal end is free. In some embodiments, the second terminal end independently comprises an alkyl, ester, amine, amide, or carboxyl group. In some embodiments, the second terminal end of each side chain does not include a hydroxyl group.

In some embodiments, the terminal end of each side chain individually comprises an ester, amine, amide, alkyl, or carboxyl. In some embodiments, the terminal end of each side chain does not include a hydroxyl group. The terminal end may be modified. The terminal end may be natural or unmodified. The terminal end of each side chain may be the same or different from the terminal end of an adjacent side chain. In some embodiments, the terminal end of each side chain is the same as the terminal end of an adjacent side chain. In some embodiments, the terminal end of each side chain is different from the terminal end of an adjacent side chain.

### b. Molecule

. The molecule may include any suitable molecule whose antigenicity is to be reduced or eliminated. The molecule may be selected from a nucleotide, polynucleotide, protein, peptide, polypeptide, carbohydrate, lipid, small molecule, or a combination thereof. In some embodiments, the molecule comprises a polypeptide, a polynucleotide, a small molecule, or a combination thereof. In some embodiments, the molecule comprises one or more peptides or protein therapeutic agents. In some embodiments, the molecule comprises a polypeptide. In some embodiments, the molecule comprises a small molecule. In some embodiments, the molecule comprises a protein. In some embodiments, the molecule comprises a drug. In some embodiments, the molecule comprises a therapeutic. In some embodiments, the molecule comprises a cancer therapeutic. In some embodiments, the molecule comprises an antibody. In some embodiments, the molecule comprises exendin.

The molecule may include, for example, a monoclonal antibody, blood factor, betatrophin, exendin, enzyme, asparaginase, glutamase, arginase, arginine deaminase, adenosine deaminase (ADA), ADA-2, ribonuclease, cytosine deaminase, trypsin, chymotrypsin, papain, growth factor, epidermal growth factor (EGF), insulin, insulin-like growth factor (IGF), transforming growth factor (TGF), nerve growth factor (NGF), platelet-derived growth factor (PDGF), bone morphogenic protein (BMP), fibroblast growth factor (FGF), somatostatin, somatotropin, somatropin, somatrem, calcitonin, parathyroid hormone, colony stimulating factors (CSF), clotting factors, tumor necrosis factors (TNF), gastrointestinal peptides, vasoactive intestinal peptide (VIP), cholecystokinin (CCK), gastrin, secretin, erythropoietins, growth hormone , GRF, vasopressins, octreotide, pancreatic enzymes, superoxide dismutase, thyrotropin releasing hormone (TRH), thyroid stimulating hormone, luteinizing hormone, luteinizing hormone-releasing hormone (LHRH), growth hormone releasing hormone (GHRH), tissue plasminogen activators, interleukins, interleukin-1, interleukin-15, interleukin-2, interleukin-10, colony stimulating factor, granulocyte macrophage colony-stimulating factor (GM-CSF), interleukin-1 receptor antagonist (IL-1RA), glucagon-like peptide-1 (GLP-1), exenatide, GLP-1 R multi-agonist, GLP-1 R antagonist, GLP-2, TNF-related apoptosis-inducing ligand (TRAIL), leptin, ghrelin, granulocyte monocyte colony stimulating factor (GM-CSF), interferons, interferon-a, interferon-gamma, human growth hormone (hGH) and antagonist, macrophage activator, chorionic gonadotropin, heparin, atrial natriuretic peptide, hemoglobin, relaxin, cyclosporine, oxytocin, vaccines, monoclonal antibodies, single chain antibodies, ankyrin repeat proteins, affibodies, activin receptor 2A extracellular domain, alpha-2 macroglobulin, alpha-melanocyte, apelin, bradykinin B2 receptor antagonist, cytotoxic T-lymphocyte-associated protein (CTLA-4), elafin, Factor IX, Factor VIIa, Factor VIII, hepcidin, infestin-4, kallikrein inhibitor, L4F peptide, lacritin, parathyroid hormone (PTH), peptide YY (PYY), thioredoxin, thymosin B4, urate oxidase, urodilatin, aptamers, silencing RNA, microRNA, long non-coding RNA, ribozymes, analogs and derivatives thereof, and combinations thereof.

The molecule may include a sortase A recognition site, a His-tag, stimulus-responsive polypeptide, or a combination thereof. Stimulus-responsive polypeptides may include environmentally responsive polypeptides. The stimulus-responsive polypeptide may include, for example, an elastin-like polypeptide, a polypeptide comprising a repeated motif (as disclosed in, for example, US 2015/0112022, filed December 16, 2014), or a resilin-like polypeptide, or a combination thereof. In some embodiments, the molecule comprises a polypeptide comprising a sortase A recognition site. In some embodiments, the sortase A recognition site comprises LPXTG (SEQ ID NO: 1), wherein X is any amino acid.

The branched polymer may be conjugated to any site anywhere on the molecule. For example, when the molecule comprises a polypeptide, the branched polymer may be conjugated to the polypeptide at the C-terminus, the N-terminus, or an internal amino acid, or a combination thereof. In some embodiments, the molecule comprises a polypeptide with the branched polymer conjugated to the C-terminus of the polypeptide. One branched polymer may be conjugated to the molecule. More than one branched polymer may be conjugated to the molecule, each branched polymer conjugated to a different site of the molecule. In some embodiments, the molecule comprises a polypeptide, and wherein more than one branched polymer is conjugated to the polypeptide, each branched polymer conjugated to a different site of the polypeptide selected from the C-terminus, the N-terminus, an internal amino acid, or a combination thereof. At least about 50%, at least about 55%, at least about 60%, at least about 65%, at least about 70%, at least about 75%, at least about 80%, at least about 85%, at least about 90%, or at least about 95% of the polypeptides have a conjugated polymer initiated solely from the C-terminus. In some embodiments, at least about 50% of the polypeptides have a conjugated polymer initiated solely from the C-terminus. In some embodiments, at least about 75% of the polypeptides have a conjugated polymer initiated solely from the C-terminus. In some embodiments, at least about 90% of the polypeptides have a conjugated polymer initiated solely from the C-terminus.

### c. Conjugate Properties

The conjugates according to the invention have reduced antigenicity or eliminated antigenicity compared to a control comprising the molecule conjugated to unbranched PEF. The conjugates may have reduced opsonization of the molecule, a reduced immune response, lack of reactivity with pre-existing anti-PEG antibodies in a subject, an *in vivo* half-life that is at least 25% greater, or an *in vivo* biodistribution to a tissue, organ, or disease site that is at least 25% greater, compared to a control..

In some embodiments, the conjugates have an *in vivo* half-life that is at least 25% greater compared with the *in vivo* half-life of the molecule alone or other molecule-polymer conjugates; or an *in vivo* biodistribution to a tissue, organ, or disease site that is at least 25% greater than the *in vivo* biodistribution of the molecule alone or other molecule-polymer conjugates. According to the invention, the antigenicity of the conjugate is reduced compared to the molecule conjugated to unbranched PEG. In some embodiments, the conjugates have reduced antigenicity compared to other molecule-polymer conjugates. In some embodiments, the conjugates have reduced binding to anti-PEG antibodies compared to other molecule-polymer conjugates or molecules. In some embodiments, the conjugates induce a reduced immune response compared to other molecule-polymer conjugates or molecules. The conjugates according to the invention are not reactive with pre-existing anti-PEG antibodies in a subject. In some embodiments, the conjugates have an *in vivo* half-life that is at least 25% greater compared with the *in vivo* half-life of the molecule. In some embodiments, the conjugates have an *in vivo* biodistribution to a tissue, organ, or disease site that is at least 25% greater than the *in vivo* biodistribution of the molecule. In some embodiments, the conjugates have an *in vivo* half-life that is at least 80% greater than the *in vivo* half-life of the molecule. The branched polymer comprises POEGMA, and the molecule-polymer conjugate is not reactive with pre-existing anti-PEG antibodies in a subject.

The methods detailed herein may enable control over site and stoichiometry of conjugation of the branched polymer to the molecule. The methods detailed herein may enable a high degree of molecular weight tunability and low dispersity of the branched polymer conjugated to the molecule, which may translate to a more predictable therapeutic performance relative to other polymer conjugates of therapeutic biomolecules. The molecule-polymer conjugates detailed herein may be more homogenous than conventional PEGylated molecules, in terms of the conjugation site, the molecular weight of the branched polymer, or a combination thereof.

The molecule-polymer conjugates detailed herein may facilitate less frequent administration, prevent an undesirable peak-to-valley fluctuation of the drug concentration *in vivo,* increase patient compliance, and reduced treatment cost, or a combination thereof.

### 3. Synthesis Of The Conjugate

Methods of making the conjugate may include, for example, those detailed in International Patent Application No. PCT/US2014/040319, filed May 30, 2014, published as WO 2014/194244.

In some embodiments, the molecule is conjugated to the backbone of the branched polymer via a linker. The molecule may be conjugated to the backbone of the branched polymer via more than one linker. The molecule may be conjugated to the backbone of the branched polymer via at least 1, at least 2, at least 3, at least 4, at least 5, at least 6, at least 7, at least 8, at least 9, or at least 10 linkers. The molecule may be conjugated to the backbone of the branched polymer via less than 20, less than 15, less than 10, or less than 5 linkers. The molecule may be conjugated to the backbone of the branched polymer via between 1 and 20, between 5 and 15, or between 1 and 5 linkers. The linker may be a polypeptide of any amino acid sequence and length. The linker may act as a spacer peptide. In some embodiments, the linker comprises charged amino acids. In some embodiments, the linker comprises uncharged amino acids. In some embodiments, the linker is flexible. In some embodiments, the linker comprises one or more cysteines. In some embodiments, the linker comprises an amino acid sequence selected from SEQ ID NO: 4 (GGC), SEQ ID NO: 5 ((GGC)₈), SEQ ID NO: 6 ((G₄S)₃), and SEQ ID NO: 7 ((VPGXG)₁₆ wherein X is valine or cysteine present in a ratio of 1:1). The linker may serve as an attachment site for the molecule to the branched polymer. The molecule may attach to the linker by any suitable means known in the art. The molecule may attach to the linker through a thiol reactive linking group. In some embodiments, the molecule is attached to one or more branched polymers via the linker. In some embodiments, the molecule is attached to the branched polymer through a thiol reactive group in the linker.

The conjugate may be made by joining or conjugating a branched polymer to a polypeptide with a sortase. In some embodiments, the molecule comprises a polypeptide that includes a sortase A recognition site, and the branched polymer and the polypeptide are incubated with Sortase A under conditions to conjugate the branched polymer to the sortase A recognition site of the polypeptide. In some embodiments, the conjugating includes contacting the molecule with a sortase A and an initiator agent under conditions that permit attachment of the initiator agent to the sortase A recognition site to form a macroinitiator, and incubating the macroinitiator with a monomer under conditions that permit free-radical polymerization and formation of a branched polymer to occur from the initiator agent to form the molecule-polymer conjugate. In some embodiments, the macroinitiator and monomer are incubated with a catalyst. The monomer may include at least one of an acrylate, methacrylate, acrylamide, and methacrylamide.

In some embodiments, the branched polymer is synthesized and subsequently grafted to the molecule to form the molecule-polymer conjugate. In some embodiments, the branched polymer is synthesized using free-radical polymerization. In some embodiments, the branched polymer is synthesized using at least one method selected from ionic ring-opening polymerization (ionic ROP), ring opening metathesis polymerization, ionic polymerization, condensation polymerization, and coordination polymerization.

In some embodiments, the free-radical polymerization comprises at least one of atom transfer radical polymerization (ATRP), reversible addition-fragmentation chain transfer (RAFT), radical ring-opening polymerization (radical ROP), nitroxide-mediated radical polymerization (NMP), iniferter polymerization, free radical polymerization, cobalt-mediated radical polymerization, telluride-mediated polymerization, and stibine-mediated polymerization.

The yield of molecule-polymer conjugates may be at least about 50%, at least about 55%, at least about 60%, at least about 65%, at least about 70%, at least about 75%, at least about 80%, at least about 85%, at least about 90%, or at least about 95%. In some embodiments, the yield of molecule-polymer conjugates is at least about 75%. In some embodiments, the yield of molecule-polymer conjugates is at least about 85%.

In some embodiments wherein the molecule comprises a polypeptide, at least about 20% of the polypeptides have a conjugated branched polymer solely at the C-terminus. At least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, or at least about 95% of the polypeptides have a conjugated branched polymer solely at the C-terminus.

In some embodiments, the molecule-polymer conjugates are separated by chromatography, such as size-exclusion chromatography, ion exchange chromatography, affinity chromatography, or hydrophobic interaction chromatography, or a combination thereof. In some embodiments, the chromatography comprises size-exclusion chromatography.

### 4. Administration

A composition may comprise the conjugate. The conjugates as detailed above can be formulated into a composition in accordance with standard techniques well known to those skilled in the pharmaceutical art. The composition may be prepared for administration to a subject. Such compositions comprising a conjugate can be administered in dosages and by techniques well known to those skilled in the medical arts taking into consideration such factors as the age, sex, weight, and condition of the particular subject, and the route of administration.

The conjugate can be administered prophylactically or therapeutically. In prophylactic administration, the conjugate can be administered in an amount sufficient to induce a response. In therapeutic applications, the conjugates are administered to a subject in need thereof in an amount sufficient to elicit a therapeutic effect. An amount adequate to accomplish this is defined as "therapeutically effective dose." Amounts effective for this use will depend on, e.g., the particular composition of the conjugate regimen administered, the manner of administration, the stage and severity of the disease, the general state of health of the patient, and the judgment of the prescribing physician.

The conjugate can be administered by methods well known in the art as described in Donnelly et al. (Ann. Rev. Immunol. 1997, 15, 617-648); Felgner et al. (U.S. Patent No. 5,580,859, issued Dec. 3, 1996); Felgner (U.S. Patent No. 5,703,055, issued Dec. 30, 1997); and Carson et al. (U.S. Patent No. 5,679,647, issued Oct. 21, 1997).

The conjugate can be complexed to particles or beads that can be administered to an individual, for example, using a vaccine gun. One skilled in the art would know that the choice of a pharmaceutically acceptable carrier, including a physiologically acceptable compound, depends, for example, on the route of administration.

The conjugates can be delivered via a variety of routes. Typical delivery routes include parenteral administration, e.g., intradermal, intramuscular or subcutaneous delivery. Other routes include oral administration, intranasal, intravaginal, transdermal, intravenous, intraarterial, intratumoral, intraperitoneal, and epidermal routes. The conjugate may be administered intravenously, intraarterially, or intraperitoneally to the subject.

The conjugate can be a liquid preparation such as a suspension, syrup, or elixir. The conjugate can be incorporated into liposomes, microspheres, or other polymer matrices (such as by a method described in Felgner et al., U.S. Patent No. 5,703,055; Gregoriadis, Liposome Technology, Vols. I to III (2nd ed. 1993). Liposomes can consist of phospholipids or other lipids, and can be nontoxic, physiologically acceptable and metabolizable carriers that are relatively simple to make and administer.

The conjugate may be used as a vaccine. The vaccine can be administered via electroporation, such as by a method described in U.S. Patent No. 7,664,545. The electroporation can be by a method and/or apparatus described in U.S. Patent Nos. 6,302,874; 5,676,646; 6,241,701; 6,233,482; 6,216,034; 6,208,893; 6,192,270; 6,181,964; 6,150,148; 6,120,493; 6,096,020; 6,068,650; and 5,702,359. The electroporation can be carried out via a minimally invasive device.

The conjugate may be administered in a controlled release formulation. The conjugate may be released into the circulation or a tumor, for example. The conjugate may be released over a period of at least about 1 day, at least about 2 days, at least about 3 days, at least about 4 days, at least about 5 days, at least about 6 days, at least about 7 days, at least about 1 week, at least about 1.5 weeks, at least about 2 weeks, at least about 2.5 weeks, at least about 3.5 weeks, at least about 4 weeks, or at least about 1 month.

### 5. Methods

### a. Methods Of Reducing The Antigenicity Of A Molecule

Provided herein are methods of reducing the antigenicity of a molecule. The methods include conjugating at least one branched polymer to a molecule to form a molecule-polymer conjugate, as detailed herein.

In some instances, the molecule comprises a polypeptide comprising a sortase A recognition site, and the branched polymer and the polypeptide are incubated with sortase A under conditions to conjugate the branched polymer to the sortase A recognition site of the polypeptide. In some embodiments, the molecule comprises a polypeptide comprising a sortase A recognition site, and the conjugating includes (a) contacting the molecule with a sortase A and an initiator agent under conditions that permit attachment of the initiator agent to the sortase A recognition site to form a macroinitiator; and (b) incubating the macroinitiator with a monomer under conditions that permit free-radical polymerization and formation of a branched polymer to occur from the initiator agent to form the molecule-polymer conjugate. In some embodiments, the sortase A recognition site comprises LPXTG (SEQ ID NO: 1), wherein X is any amino acid. In some embodiments, the macroinitiator and monomer are incubated with a catalyst in step (b). In some instances not presently claimed, the monomer in step (b) comprises at least one of an acrylate, methacrylate, acrylamide, and methacrylamide. In some embodiments, the method further includes separating the molecule-polymer conjugate formed in step (b) from the unreacted macroinitiator.

The methods may further include separating the molecule-polymer conjugate formed in step (b) from the unreacted macroinitiator. In some embodiments, the yield of molecule-polymer conjugate is at least about 50% of the total conjugates and macroinitiators which are separated. In some embodiments, the molecule-polymer conjugate is separated by chromatography. In some embodiments, chromatography comprises size-exclusion chromatography, ion exchange chromatography, affinity chromatography, or hydrophobic interaction chromatography, or a combination thereof. In some embodiments, the chromatography comprises size-exclusion chromatography.

In some embodiments, the branched polymer is synthesized and subsequently grafted to the molecule to form the molecule-polymer conjugate. In some embodiments, the branched polymer is synthesized using free-radical polymerization. In some embodiments, the branched polymer is synthesized using at least one method selected from ionic ring-opening polymerization (ionic ROP), ring opening metathesis polymerization, ionic polymerization, condensation polymerization, and coordination polymerization.

In some embodiments, conjugating at least one branched polymer to a molecule to form a molecule-polymer conjugate comprises attaching an initiator agent to the molecule to form a macroinitiator; and incubating the macroinitiator with a monomer under conditions that permit free-radical polymerization and formation of a branched polymer to occur from the initiator agent to form the molecule-polymer conjugate. In some embodiments, the macroinitiator and monomer are incubated with a catalyst. In some instances not presently claimed, the monomer comprises at least one of an acrylate, methacrylate, acrylamide, and methacrylamide. In some embodiments, the method further includes separating the molecule-polymer conjugate from the unreacted macroinitiator.

In some embodiments, the free-radical polymerization comprises at least one of atom transfer radical polymerization (ATRP), reversible addition-fragmentation chain transfer (RAFT), radical ring-opening polymerization (radical ROP), nitroxide-mediated radical polymerization (NMP), iniferter polymerization, free radical polymerization, cobalt-mediated radical polymerization, telluride-mediated polymerization, and stibine-mediated polymerization.

### b. Methods Of Making Molecule-Polymer Conjugates

Provided herein, but not presently claimed, are methods of making molecule-polymer conjugates having reduced or eliminated antigenicity compared to a control. The molecule may include a polypeptide having a sortase A recognition site. The method may include (a) contacting the molecule with a sortase A and an initiator agent under conditions that permit attachment of the initiator agent to the sortase A recognition site to form a macroinitiator; and (b) incubating the macroinitiator with a monomer under conditions that permit free-radical polymerization to occur from the initiator agent to form the molecule-polymer conjugate.

The sortase A recognition site may comprise LPXTG (SEQ ID NO: 1), wherein X is any amino acid. The macroinitiator and monomer may be incubated with a catalyst in step (b). The monomer in step (b) may comprise at least one of an acrylate, methacrylate, acrylamide, and methacrylamide. The free-radical polymerization may comprise at least one of atom transfer radical polymerization (ATRP), reversible addition-fragmentation chain transfer (RAFT), radical ring-opening polymerization (radical ROP), nitroxide-mediated radical polymerization (NMP), iniferter polymerization, free radical polymerization, cobalt-mediated radical polymerization, telluride-mediated polymerization, and stibine-mediated polymerization. The free-radical polymerization may comprise at least one of ionic ring-opening polymerization (ionic ROP), ring opening metathesis polymerization, ionic polymerization, condensation polymerization, and coordination polymerization.

The free-radical polymerization may comprise at least one of atom transfer radical polymerization (ATRP), reversible addition-fragmentation chain transfer (RAFT), radical ring-opening polymerization (radical ROP), nitroxide-mediated radical polymerization (NMP), iniferter polymerization, free radical polymerization, cobalt-mediated radical polymerization, telluride-mediated polymerization, and stibine-mediated polymerization.

The methods may further include separating the molecule-polymer conjugate formed in step (b) from the unreacted macroinitiator. The yield of molecule-polymer conjugate may be at least about 50% of the total conjugates and macroinitiators which are separated. The molecule-polymer conjugate may be separated by chromatography. Chromatography may comprise size-exclusion chromatography, ion exchange chromatography, affinity chromatography, or hydrophobic interaction chromatography, or a combination thereof. Chromatography may comprise size-exclusion chromatography.

### 6. Examples

### Example 1

### Materials and Methods

***Experimental Design.*** All *in vitro* and *in vivo* experiments include suitable controls; where applicable, PBS served as a negative control and unmodified exendin served as a positive control. The sample sizes for *in vivo* studies were chosen based on similar studies conducted previously (Amiram, M., et al. Proc. Natl. Acad. Sci. 2013, 110, 2792-2797; Schellenberger, V., et al. Nat. Biotechnol. 2009, 27, 1186-1188). See *Animal studies* section below for details on the animal model used. Mice were randomly grouped before initiation of each experiment. The investigator was not blinded to group allocation. For the *in vivo* fed glucose measurement studies, mouse blood glucose levels were measured until all experimental groups no longer showed statistical significance in glucose reduction compared to the PBS control group. All collected data points were included in data analysis.

***Cloning, expression and purification.*** All molecular biology reagents were purchased from New England Biolabs unless otherwise specified. The gene encoding exendin in a pMA-T vector was codon optimized and synthesized by Life Technologies. The first methionine residue encoding the translational start codon in proteins recombinantly expressed in *E. coli* needs to be cleaved post-translationally for proper function and stability of the protein. However, the first amino acid of exendin is a histidine, and our past experience and reports in the literature both suggest that having histidine as the residue immediately following methionine prevents proper methionine cleavage. Thus, a di-alanine leader was incorporated at the N-terminus of the peptide to facilitate methionine cleavage. Once *in vivo,* the di-alanine leader can be cleaved by dipeptidyl peptidase 4 (DPP4), an exopeptidase that cleaves N-terminal dipeptides containing proline or alanine as the second residue, to reveal the N-terminus of exendin for GLP-1R binding. The exendin gene was amplified by polymerase chain reaction (PCR), using forward and reverse primers containing Ndel overhangs and with the sequence for the sortase A recognition motif "LPETG" (named "srt" for brevity) followed by a His₆-tag incorporated in the reverse primer. The amplified "exendin-srt-His₆" fragment was inserted into a modified pET-24a+ vector at an Ndel restriction site immediately upstream of an ELP with the sequence (VPGVG)₆₀, to yield "exendin-srt-His₆-ELP".

Expression and purification of the quaternary fusion protein followed previously described procedures with minor changes (Qi, Y., et al. Macromol. Rapid Commun. 2013, 34, 1256-1260). Briefly, cells were cultured in Terrific Broth (TB, Mo Bio Laboratories, Inc.) supplemented with 45 µg/mL of kanamycin at 25°C. Once the optical density at 600 nm (OD₆₀₀) of the culture reached 0.6, temperature was lowered to 16°C and isopropyl β-D-1-thiogalactopyranoside (IPTG, AMRESCO) was added to a final concentration of 0.1 mM to induce protein expression. Cells were harvested 15 h post induction by centrifugation at 700×g for 10 min and were lysed by sonication on a Misonex Ultrasonic Liquid Processer (Qsonica, LLC.) at amplitude 85 for 3 min with 10 sec on and 40 sec off cycles. Nucleic acids were removed from the crude extract by addition of 1 vol % polyethyleneimine (PEI, Acros) followed by centrifugation at 4°C and 21,000xg for 10 min. The ELP tag enables purification of the fusion protein by ITC, a nonchromatographic method that we have previously developed for the purification of ELP fusion proteins that takes advantage of the inverse phase transition behavior imparted by the ELP (Meyer, D. E. & Chilkoti, A. Nat. Biotechnol. 2009, 14, 1112-1115). After triggering the inverse phase transition of the fusion by addition of 0.1 M ammonium sulfate, the aggregated proteins were collected by centrifugation at ~30°C and 21,000xg for 10 min. The pellet was then resolubilized in cold PBS and the resulting solution was centrifuged at 4°C and 21,000xg for 10 min to remove any remaining insoluble material. The last two steps were typically repeated one more time to obtain homogeneous protein, as verified by SDS-PAGE. In the final step, the protein was resolubilized in sortase buffer (50 mM Tris, 150 mM NaCl, 10 mM CaCl₂, pH adjusted to 7.5) in preparation for sortase-catalyzed initiator attachment.

The gene for sortase A with a 59 N-terminal amino acid truncation (previously shown to not affect its transpeptidase activity) and an N-terminal His6-tag in a pET15b vector was available from a previous study. Expression and purification of His₆-sortase A were carried out as previously described (Qi, Y., et al. Macromol. Rapid Commun. 2013, 34, 1256-1260).

***Sortase-catalyzed initiator attachment and macroinitiator purification.*** The exendin-C-Br macroinitiator was synthesized and purified following procedures described previously with minor changes (Qi, Y., et al. Macromol. Rapid Commun. 2013, 34, 1256-1260). Briefly, a reaction mixture consisting of exendin-srt-His₆-ELP, His₆-sortase A, and AEBMP at a 2:1:60 ratio in sortase buffer was incubated at 20°C for 18 h. After reaction, a reverse His-tag purification was used to isolate the exendin-C-Br macroinitiator, by exploiting the fact that it is the only species in the mixture without a His₆-tag. Purification was performed on an AKTA Purifier (GE Healthcare) equipped with a photodiode detector set at 280 nm and a HisTrap HP column. Elution through the column with PBS yielded pure exendin-C-Br in the eluent while leaving all other unwanted species bound to the resin. The collected exendin-C-Br was dialyzed overnight in PBS (pH 7.4) to remove residual free initiator.

***Macroinitiator characterization.*** MALDI-MS was performed on a Voyager-DE Pro mass spectrometer (Life Technologies). Samples at -25 µM in PBS were diluted 1:10 with 10 mg/mL sinapinic acid in 90:10 water/acetonitrile with 0.1 vol % trifluoroacetic acid (TFA) as the ionization matrix. The instrument was operated in linear mode with positive ions generated using a N₂ laser. Ubiquitin was used as a molecular weight standard to calibrate the instrument.

For LC/MS-MS analysis to confirm site-specificity of initiator attachment, 100 µL of ~8 uM exendin-C-Br in PBS was solvent exchanged into 50 mM ammonium bicarbonate (pH 8.0) on a ZebaSpin desalting column (Thermo Fisher Scientific) followed by trypsin (sequencing grade, Promega) digestion at 37°C for 18 h directly in the column. The digestion mixture was collected by centrifugation, dried by vacuum centrifugation and was then resuspended in 20 µL 2% acetonitrile and 0.1% formic acid in water. 1 µL of the sample was separated on a NanoAquity ultra performance liquid chromatography (UPLC, Waters) system equipped with a BEH130 C18 reversed phase column (Waters) using a mobile phase consisting of (A) 0.1% formic acid in water and (B) 0.1% formic acid in acetonitrile. A linear gradient of 5% B to 40% B was performed over 60 min at 400 nL/min and the separated peptides were ionized by electrospray ionization (ESI) followed by MS analysis on a Synapt G2 HDMS QToF mass spectrometer (Waters). The top four most abundant ions were selected for MS/MS. Mass spectra were processed with Mascot Distiller (Matrix Science) and were then submitted to Mascot searches (Matrix Science) against a SwissProt_Ecoli database appended with the custom exendin-C-Br sequence. Search results were imported into Scaffold (v4.0, Proteome Software) and scoring thresholds were set to yield a minimum of 99% protein confidence for protein identification. Extracted ion chromatograms were performed in MassLynx (v4.1). Experimental isotope distributions of the brominated C-terminal tryptic peptide was compared to a theoretical isotope distribution modeled in Molecular Weight Calculator (v. 6.49, Pacific Northwest National Laboratory, ncrr.pnl.gov/software).

***In situ ARGET-ATRP.*** All chemical reagents were purchased from Sigma Aldrich and used as received, unless otherwise specified. EG9 OEGMA monomer (Mₙ ~500 Da or ~9 side-chain EG repeats on average, Sigma Aldrich, #447943) and EG3 OEGMA monomer (triethylene glycol methyl ether methacrylate, 232 Da, Sigma Aldrich, #729841) were passed through a column of basic alumina to remove the inhibitors.

In a typical reaction, 216 µmol of OEGMA and 21.6 µL of a stock solution of 200 mM CuBr₂ and 1.6 M tris(2-pyridylmethyl)amine (TPMA) pre-complexed in MilliQ water with 5% dimethylformamide (DMF) were mixed with 1 mL of 500 µM exendin-C-Br in PBS in a Schlenk flask. A 3.2 mM solution of ascorbic acid in MilliQ water was prepared in a separate flask. The two solutions were degassed by bubbling with argon for 30 min, after which Activator-Regenerated Electron Transfer (ARGET) ATRP was initiated and maintained by continuously injecting the ascorbic acid solution into the reaction medium using a syringe pump at a rate of 1.6 nmol/min. Polymerization was allowed to proceed for a specified time at 20°C under argon and was quenched by bubbling with air. Reactions of the EG3 OEGMA were done with 443 µmol of the monomer in 20 v/v% methanol in PBS while all other conditions remained the same. At the end of the reaction, the reaction mixture was dialyzed against PBS overnight to remove residual small molecule reagents in preparation for downstream characterization and purification.

***Characterization of OEGMA monomers.*** Monomers diluted 1:20,000 in methanol were separated on an Agilent 1100 LC system equipped with a Zorbax Eclipse Plus C18 column (Agilent) using a mobile phase consisting of (A) 0.3% formic acid in water and (B) 0.3% formic acid in acetonitrile. A linear gradient of 50% B to 95% B was performed over 10 min at 50°C. Separated samples were ionized by ESI followed by MS analysis on an Agilent MSD ion trap mass spectrometer.

***Physical characterization of exendin-C-POEGMA conjugates.*** Analytical SEC was performed on a Shimadzu high performance liquid chromatography (HPLC) system equipped with a UV-vis detector (SPD-10A VP) operating at 280 nm. 50 µL of samples at ~2 mg/mL were separated on a Protein KW-803 column (Shodex) using 0.1M Tris-HCI (pH 7.4) as mobile phase at 25°C with a flow rate of 0.5 mL/min. Conjugation efficiency of *in situ* ATRP from exendin was calculated by quantifying AUC of peaks detected at 280 nm. Sum of the AUC's of the two peaks corresponding to the unreacted macroinitiator and the conjugate in each chromatogram was regarded as 100% and % fraction of the conjugate peak was calculated as the conjugation efficiency of that particular polymerization reaction.

The fluid line of the analytical HPLC system was connected downstream in series to a DAWN HELEOS II MALS detector followed by an Optilab T-rEX refractometer (both from Wyatt Technology) for conducting SEC-MALS analysis. The system was calibrated with toluene and normalized with 2.0 mg/mL bovine serum albumin (BSA, Pierce). Samples were passed through 0.1 µm filters before injection. dn/dc values of the conjugates were determined on an Anton Paar Abbemat 500 refractometer (Anton Paar). Data were analyzed in ASTRA (v. 6.0, Wyatt Technology) to compute M_{w}, Mₙ and *Ð* of the conjugates.

Conjugates were purified by a single round of preparative SEC on an AKTA Purifier equipped with a photodiode detector set at 280 nm and a HiLoad 26/600 Superdex 200 PG column using PBS as mobile phase at 4°C and a flow rate of 2.0 mL/min.

DLS was performed on a DynaPro Plate Reader (Wyatt Technology). Samples were prepared at 25 µM and filtered with 0.1 µm filters before analysis. The instrument was operating at a laser wavelength of 831.95 nm, a scattering angle of 90° and at 25°C. Data were analyzed in Dynals mode using Dynamics 6.12.0.3.

***General biochemical analysis.*** Concentrations of fusion proteins were measured on a ND-1000 Nanodrop spectrophotometer (Thermo Scientific) by UV-vis absorption spectroscopy. Concentration of exendin and conjugates for *in vitro* assays and *in vivo* studies was assessed using a Bicinchoninic Acid (BCA, Pierce) assay following manufacturer's instructions. SDS-PAGE analysis of sortase A was performed using precast 4-20% Tris-HCI gels (Bio-Rad). SDS-PAGE analyses of all exendin derivatives were performed using precast Tris/Tricine gels (Bio-Rad). Quantification of sortase reaction conversion was done by gel densitometry analysis using a built-in function in Image Lab (v. 4.0.1, Bio-Rad).

***In vitro cAMP ELISA.*** Activity of native exendin and conjugates was assessed *in vitro* by quantifying intracellular cAMP release as a result of GLP-1R activation in BHK cells stably transfected with rat GLP-1R (a generous gift of Drucker group, University of Toronto, Toronto, Canada)(Drucker, D. J. & Nauck, M. A. Lancet 2006, 368, 1696-1705). Cells were allowed to reach 70-80% confluence in 24-well plates. Prior to the assay, -20 µg of peptide or equivalent of conjugates were treated with 0.5 µg DPP4 (ProSpect) overnight to remove the di-alanine leader. On the day of the assay, cells were incubated with 3-isobutyl-1-methylxanthineto (IBMX, EMD Millipore) for 1 h to prevent cAMP degradation, followed by incubation with varying concentrations (0.001-1000 nM in log-scale increments) of exendin (Genscript) or conjugates for 10 min to trigger GLP-1R activation. 0.1 M HCI was then added to disrupt the cells and release intracellular cAMP. cAMP concentration was measured by a competitive cAMP ELISA according to the manufacturer's protocol (Enzo Life Sciences). Each sample was assayed in triplicate and data were analyzed in Igor Pro (v. 6.2, Wavemetrics) using a Hill equation fit to determine the EC50 of each construct (Goutelle, S. et al. Fundam. Clin. Pharmacol. 2008, 22, 633-648).

***Animal studies.** In vivo* experiments were performed with 6-week-old male C57BL/6J mice (stock no. 000664) purchased from Jackson Laboratories. Upon arrival, mice were initiated on a 60 kCal% fat diet (#D12492, Research Diets Inc.) to induce a diabetic phenotype. Previous studies have established high fat-fed C57BL/6J mice as an adequate model for type 2 diabetes, as after one week on a high-fat diet, mice exhibit elevated blood glucose, progressively increasing insulin level, and severely compromised insulin response and glucose tolerance (Winzell, M. S. & Ahren, B. Diabetes 2004, 53, S215-S219; Surwit, R. S., et al. Diabetes 1988, 37, 1163-1167). Mice were housed under controlled light on a 12 h light/12 h dark cycle with free access to food and water. All mice were allowed to acclimate to the high-fat diet and the facility for 10 d before initiation of experiments. Mice used for fed glucose measurement study of EG3 conjugates were maintained on the high-fat diet for 3 weeks and used at the age of 8 weeks. All animal care and experimental procedures were approved by the Duke Institutional Animal Care and Use Committee.

***In vivo fed glucose measurements.*** The effect of native exendin and the conjugates on fed blood glucose levels was measured following a single s.c. injection of each sample. Before blood glucose measurement, the tail was wiped with a sterilizing alcohol solution and wiped dry. A tiny incision was made on the mouse tail vein using a disposable lancet, and the first 1 µL drop of blood was wiped off. The second 1-2 µL blood drop was used for glucose measurement using a hand-held glucometer (AlphaTrack, Abbott). Blood glucose levels were measured 1 d before the experiment. On the day of injection, weights and blood glucose were measured, and a sample solution or PBS control of equivalent volume was injected s.c. Immediately following injection, mice were placed back in the cage with free access to food and water, and blood glucose was measured at 1, 4, 6 (exendin only), 8, 24, 48, 72, 96, 120 and 144 h post-injection. Weights were monitored daily. In the EG9 dose-dependent study, a 66.2 kDa EG9 exendin-C-POEGMA conjugate was injected into mice (n=3) at 25, 50, and 85 nmol/kg mouse body weight. In the EG9 MW-dependent study, EG9 conjugates of 25.4, 54.6, 97.2 and 155.0 kDa Mₙs were injected into mice (n=6) at 25 nmol/kg. In the EG3 fed glucose study, 55.6kDa and 71.6 kDa EG3 exendin-C-POEGMA conjugates were injected into mice (n=5) at 25 nmol/kg. Blood glucose levels were normalized by the average glucose levels measured 24 h and immediately before injection to reflect the percent change in blood glucose and to correct for transient variations in glucose.

***In vivo IPGTT.*** Mice were randomly divided into groups (n=5 in **FIG. 4a** and **FIG. 4b**, n=3 in **FIG. 4c** and FIG. **4d**). On day one, every two groups of mice received a s.c. injection of either 54.6 kDa EG9 exendin-C-POEGMA conjugate, exendin as positive control, or PBS at equivalent volume as negative control. Exendin and the conjugate were injected at 25 nmol/kg. 18 h after injection, one group of mice in each category were fasted by removal of food for 6 h. At the end of the fast period (24 h following injection), mice were given 1.5 g /kg glucose (10 w/v % sterile glucose solution, Sigma) via i.p. injection. Blood glucose levels were monitored by nicking the tail vein and measuring the glucose level in the blood using a glucometer at 0, 20, 40, 60, 90, and 120 min after glucose administration. 66 h after injection, the remaining groups of mice were subjected to the same protocol and an IPGTT was similarly performed 72 h following injection.

***In vivo pharmacokinetics.*** Exendin, 54.6 kDa EG9, 55.6 kDa EG3 and 71.6 kDa EG3 exendin-C-POEGMA conjugates were fluorescently labeled with Alexa Fluor^{®} 488 NHS ester (Thermo Fisher Scientific) via their solvent accessible primary amines on lysine residues and the N-terminus, according to manufacturer's protocol. Unreacted free fluorophore was removed using a ZebaSpin desalting column (Thermo Fisher Scientific). Mice were randomly divided into four groups (n=3). Animals were weighed before injection. Each group of mice received a single s.c. injection of one of the labeled samples at 75 nmol/kg (45 nmol/kg fluorophore). 10 µL of blood samples were collected from the tail vein into 100 µL of a heparin solution (1kU/ml in PBS, Sigma Aldrich) at 40 s, 40 min, 2.5 h, 4.5 h, 8 h, 24 h, 48 h, 72 h, 96 h and 120 h after injection. Blood samples were centrifuged at 4°C and 20,000 xg for 10 min to extract the plasma for fluorescence reading at excitation 485 nm and emission 535 nm on a Victor multilabel plate reader (Perkin Elmer). Plasma concentrations of constructs as a function of time were fitted using a non-compartmental analysis (PK Solutions 2.0, Summit Research Services) that characterizes the absorption and elimination phases of the profiles to derive the pharmacokinetic parameters.

***In vitro anti-PEG ELISA.*** In the direct ELISA, columns of a 96-well microtiter plate (CoStar) were coated with Krystexxa^{®} (Crealta Pharmaceuticals), ADA (Sigma-Tau Pharmaceuticals), Adagen^{®} (Sigma-Tau Pharmaceuticals), exendin (Genscript), a 54.6 kDa EG9 exendin-C-POEGMA conjugate, a 55.6 kDa EG3 exendin-C-POEGMA conjugate or BSA (Sigma Aldrich). The antigen solutions for plate coating were prepared in PBS to yield ~2 µg of unmodified peptide/protein or ~5 µg of PEG/OEG in the case of polymer-modified antigens per well upon adding 50 µL to each well. The PEG/OEG contents of the polymer-modified antigens were calculated as follows: Krystexxa^{®} consists of the tetrameric uricase enzyme (125 kDa total) with 10-11 lysine side-chain amino groups on each of its four subunits reacted with 10 kDa PEG p-nitrophenyl carbonate ester, giving a PEG content of -76%. Adagen^{®} consists of ADA (40.8 kDa) with 11-17 of its side-chain amino groups on solvent-accessible lysines functionalized with 5 kDa monomethoxy succinyl PEG according to the manufacturer's specifications (Sigma-Tau Pharmaceuticals). For our calculation, we assumed 14 PEG chains per Adagen^{®} conjugate on average, giving -60% PEG content. In the case of the exendin-C-POEGMA conjugates, subtracting the poly(methyl methacrylate) backbone (-17% for EG9 POEGMA and -37% for EG3 POEGMA) gives an OEG content of -75% for the 54.6 kDa EG9 conjugate and -58% for the 55.6 kDa EG3 conjugate. After overnight incubation of the coated plate at 4°C, it was washed with PBS and all wells were blocked with 1% BSA in PBS. One patient plasma sample previously tested negative for PEG antibody and two that were tested positive were diluted 1:400 v/v in 1% BSA in PBS. The two positive patient plasma samples were from two different individuals that developed anti-PEG antibodies during a Phase II clinical trial of Krystexxa^{®}. Following another round of PBS washing, 100 µL of each diluted plasma sample and 1% BSA in PBS were added to replicate wells of each antigen. The plate was then incubated at room temperature for 2 h. Wells were again washed with PBS and 100 µL of alkaline phosphatase-conjugated goat anti-human IgG (Sigma) diluted 1:5250 with 1% BSA in PBS was added to each well. After 1 h incubation at room temperature, wells were washed with PBS followed by Tris-buffered saline. Bound alkaline phosphatase was detected by incubating with p-nitrophenyl phosphate (Sigma) in accordance with the directions of the supplier. The phosphatase reaction was stopped by adding 50 µL/well of 10% NaOH, and the absorbance at 405 nm was measured on a plate reader (Tecan Infinite M200 Pro, Tecan Austria).

In the competitive ELISA, a microtiter plate was coated with 50 µL of 100 µg/mL Krystexxa^{®} per well by overnight incubation at 4°C. Various amounts of ADA, Adagen^{®}, exendin, a 54.6 kDa EG9 exendin-C-POEGMA conjugate, and a 55.6 kDa EG3 exendin-C-POEGMA conjugate were diluted with PBS to yield 0, 0.5, 2, 5, 10 and 20 µg of competing antigen per well upon adding 50 µL to each well. Dilutions of Adagen^{®} and the exendin-C-POEGMA conjugates were prepared such that at each competing antigen concentration, similar PEG/OEG contents were compared as shown in **TABLE 5**. The diluted competing antigens were mixed with equal volume of a patient plasma sample that tested positive for PEG antibody (diluted 1:200 v/v in 1% BSA in PBS) and incubated at 4°C overnight. The following morning, after washing with PBS, all wells were blocked with 1% BSA in PBS. Wells were washed with PBS after blocking, and 100 µL of each concentration of the competing antigen-plasma mixtures was added in replicate wells. After incubation at room temperature for 2 h, alkaline phosphatase-conjugated IgG was added for colorimetric readout at 405 nm as described above.

**TABLE 5**. Variable amounts of Adagen^{®} and exendin-C-POEGMA conjugates and their corresponding PEG/OEG contents loaded as competing antigens per well in the competitive ELISA. PEG content of Adagen^{®} was approximated by assuming 14 PEG chains per Adagen^{®} conjugate, while OEG content of the exendin-C-POEGMA conjugates was directly calculated by subtracting the poly(methyl methacrylate) backbone.

| Nominal (µg/well) | Adagen^{®} | | 54.6 kDa EG9 exendin-C-POEGMA | | 55.6 kDa EG3 exendin-C-POEGMA | |
|---|---|---|---|---|---|---|
| | Conjugate (µg/well) | PEG (µg/well) | Conjugate (µg/well) | OEG (µg/well) | Conjugate (µg/well) | OEG (µg/well) |
| 0.5 | 0.6 | 0.4 | 0.5 | 0.4 | 0.7 | 0.4 |
| 2 | 2.6 | 1.6 | 2.0 | 1.5 | 2.8 | 1.6 |
| 5 | 6.4 | 3.8 | 5.0 | 3.8 | 6.9 | 4 |
| 10 | 12.8 | 7.7 | 10.0 | 7.5 | 13.8 | 8 |
| 20 | 25.6 | 15.4 | 20.0 | 15.0 | 27.6 | 16 |

Assays in **FIG. 5a** and **FIG. 5b** were performed with n=3, while those in **FIG. 5c** and **FIG. 5d** were performed with n=5.

*Statistical analysis.* Data are presented as means ± standard errors (SEs). Blood glucose levels in fed glucose measurement studies (n=6) were normalized by the average glucose levels measured 24 h and immediately before injection. Treatment effects on fed glucose levels were analyzed using repeated measures two-way ANOVA, followed by *post hoc* Dunnett's multiple comparison test to evaluate individual differences between a treatment and PBS control at each time point. AUCs of fed glucose profiles were compared using one-way ANOVA followed by *post hoc* Tukey's multiple comparison test (n=6). For evaluating AUC of IPGTT (n=5), treatment and PBS were compared using an unpaired parametric two-tailed t test. Both direct and competitive anti-PEG ELISAs (n=3) were analyzed using two-way ANOVA, followed by *post hoc* Dunnett's multiple comparison test to evaluate individual differences between exendin-C-POEGMA and the other groups for each plasma sample (direct) or antigen concentration (competitive). A test was considered significant if the P value was less than 0.05. Statistical analyses were performed using Prism 6 (GraphPad software Inc.).

### Example 2 (Reference Example) Sortase-catalyzed C-terminal initiator attachment to exendin

We exploited the C-terminal native peptide ligation mechanism of sortase A to site-specifically attach the ATRP initiator N-(2-(2-(2-(2-aminoacetamido)acet-amido)acetamido) ethyl)-2-bromo-2-methylpropanamide (AEBMP) to the C-terminus of exendin (**FIG. 1**). A quaternary fusion protein, abbreviated as "exendin-srt-His₆-ELP", was recombinantly expressed to serve as the sortase A substrate (**FIG. 1a****)**. As explained in an earlier study, "srt" stands for the native sortase A recognition sequence "LPETG" and ELP refers to a stimulus-responsive elastin-like polypeptide that was incorporated to enable easy purification of the fusion protein by inverse transition cycling (ITC, **FIG. 7a**), a nonchromatographic protein purification method that we previously developed (Meyer, D. E. & Chilkoti, A. Nat. Biotechnol. 1999, 14, 1112-1115). The recognition sequence was deliberately located between the protein and the ELP, so that transpeptidation by sortase A not only attaches the initiator to exendin, but also conveniently liberates the purification tag. Sortase A with an N-terminal hexahistidine tag (His₆-tag) was recombinantly expressed from a plasmid constructed in the earlier study and was purified by immobilized metal affinity chromatography (IMAC, **FIG. 7b**). The ATRP initiator AEBMP (**FIG. 1**) was chemically synthesized with an N-terminal (Gly)₃ motif serving as the nucleophile, as maximum reaction rates for sortase-catalyzed C-terminal ligation have been reported with two or more glycines (Mao, H., et al. J. Am. Chem. Soc. 2004, 126).

Successful sortase-catalyzed initiator attachment (**FIG. 1b**) resulted in cleavage of exendin-LPETG-His₆-ELP into exendin-LPET and G-His₆-ELP, followed by attachment of AEBMP to exendin-LPET to generate the macroinitiator product (exendin-C-Br). Sodium dodecyl sulfate-polyacrylamide gel electrophoresis (SDS-PAGE) analysis of the reaction mixture (**FIG. 2a**) showed > 90% conversion to exendin-C-Br, as assessed by gel densitometry. Similar to the previous study, a His₆-tag was intentionally inserted between "srt" and ELP on the exendin-srt-His₆-ELP fusion, such that upon transpeptidation by His₆-sortase A, all the residual reactants, enzyme and side-products except the desired product ―exendin-C-Br― carried a His₆-tag. Consequently, elution through an IMAC column yielded pure exendin-C-Br (**FIG. 2a**) in the eluent while leaving all other unwanted species bound to the resin.

### Example 3

### Synthesis and characterization of exendin-C-POEGMA conjugates

Next, *in situ* Activator Regenerated by Electron Transfer (ARGET) ATRP (Jakubowski, W. & Matyjaszewski, K. Angew. Chem. Int. Ed. 2006, 45, 4482-4486) was carried out to graft POEGMA from exendin-C-Br (**FIG. 1c**). An OEGMA monomer with an average mass of ~500 Da or ~9 side-chain EG repeats (EG9) was used, as shown by liquid chromatography electrospray ionization mass spectrometry (LC/ESI-MS) analysis (**FIG. 8a**). The reaction time was varied to produce EG9 exendin-C-POEGMA conjugates with a range of MWs. Size exclusion chromatography (SEC) analysis of exendin-C-Br before polymerization detected by UV-vis absorbance at 280 nm (**FIG. 2b**) showed a single peak eluting at 23.7 min. After polymerization, the intensity of the macroinitiator peak greatly diminished, and was accompanied by the appearance of peaks at 21.3, 19.5, 17.8, 16.5, and 15.0 min, corresponding to EG9 exendin-C-POEGMA conjugates with increasing MWs as the reaction time was increased. The results from UV-vis detection were in agreement with those from refractive index (RI) detection (**FIG. 9a**). Integration of peak areas in the UV-vis chromatograms showed that the average conjugation yield was ~ 80%. As shown in **TABLE 1**, the synthesized conjugates had Mₙs that ranged from 25.4 to 155.0 kDa and all conjugates had very narrow dispersities (*Ð* ≤ 1.15). The conjugates could be easily and completely purified by a single round of preparative SEC (**FIG. 9b**).

**TABLE 1**. Physical properties and biological activity of exendin and exendin-C-POEGMA conjugates. MWs and *Ð*s were determined by size exclusion chromatography multi-angle light scattering (SEC-MALS). Rₕs were measured by dynamic light scattering (DLS). EC₅₀ values of EG9 and EG3 conjugates were derived from cAMP response curves in **FIG. 2c** and **FIG. 13**, respectively. Rₕ and EC₅₀ values are reported as mean ± SEM, n=10 for Rₕ and n=3 for EC₅₀. M_{w}: weight-average MW, Mₙ: number-average MW, *Ð*: dispersity, Rₕ: hydrodynamic radius, EC₅₀: half-maximal effective concentration. ^{a}Calculated from amino acid sequence. ^{b}Default value due to unimolecular nature of the peptide.

| **Species** | **Reaction time (h)** | **M_{w} (Da)** | **Mₙ (Da)** | ***Ð* (M_{w}/ Mₙ)** | **Rₕ (nm)** | **EC₅₀ (nM)** |
|---|---|---|---|---|---|---|
| exendin | -- | -- | 4,186.6^{a} | 1.00^{b} | 2.2 ± 0.1 | 0.08 ± 0.01 |
| EG9 | 0.5 | 26,400 | 25,400 | 1.04 | 4.5 ± 0.4 | 0.84 ± 0.09 |
| EG9 | 1 | 56,800 | 54,600 | 1.04 | 5.6 ± 0.5 | 1.91 ± 0.35 |
| EG9 | 1.25 | 72,200 | 66,200 | 1.09 | 5.9 ± 0.5 | 2.10 ± 0.08 |
| EG9 | 2 | 100,000 | 97,200 | 1.03 | 6.8 ± 0.7 | 6.67 ± 0.21 |
| EG9 | 3 | 178,000 | 155,000 | 1.15 | 7.6 ± 0.5 | 7.69 ± 0.04 |
| EG3 | 3 | 27,400 | 26,300 | 1.04 | 3.8 ± 0.4 | 3.29 ± 0.27 |
| EG3 | 5.5 | 60,600 | 55,600 | 1.09 | 4.8 ± 0.5 | 4.17 ± 0.13 |
| EG3 | 8 | 82,700 | 71,600 | 1.16 | 5.4 ± 0.6 | 5.11 ± 0.23 |

Exendin acts by binding and activating the G protein-coupled GLP-1 receptor (GLP-1R), which results in the release of cyclic adenosine monophosphate (cAMP) as a second messenger in a downstream signaling cascade, ultimately leading to secretion of insulin to regulate blood glucose. The potency of native exendin and the EG9 exendin-C-POEGMA conjugates were next assessed by quantifying intracellular cAMP release as a result of GLP-1R activation in baby hamster kidney (BHK) cells that were stably transfected with rat GLP-1R. As shown in **FIG. 2c** and **TABLE 1**, grafting EG9 POEGMA from exendin increases the EC₅₀ of the peptide in an overall MW-dependent manner, which indicates decreased receptor binding with increasing polymer MW as a result of the steric hindrance imposed by the appended POEGMA chain. Of the prepared conjugates, only those with three ethylene glycol monomers in the side chain, but not those with nine ethylene glycol monomers in the side chain, are according to the invention.

### Example 4 (Reference Example)

### In vivo therapeutic efficacy of EG9 exendin-C-POEGMA

The *in vivo* efficacy of EG9 exendin-C-POEGMA conjugates was assessed in male C57BL/6J mice that were maintained on a 60 kCal% fat diet, so as to develop a diabetic phenotype (Winzell, M. S. & Ahren, B. Diabetes 2004, 53, S215-S219; Surwit, R. S., et al. Diabetes 1988, 37, 1163-1167). A dose-dependent study was first performed to determine an adequate dose. A 66.2 kDa EG9 exendin-C-POEGMA conjugate was administered into mice via a single s.c. injection at 25, 50 and 85 nmol/kg mouse body weight of the conjugate. Fed blood glucose levels measured at various time points post-injection revealed an overall slight increase in the duration of glucose reduction with increasing dose of the conjugate compared to phosphate buffered saline (PBS) control (**FIG. 10a** and **FIG. 10b**, **TABLE 3**). A similar trend was observed in the mouse body weights, where the mice treated with the highest dose showed considerably more weight loss than the two lower doses (**FIG. 10c**). While the weight-lowering benefit of exendin has been well established, overdosing can cause nausea, which can lead to acute weight loss in rodents. The excessive weight loss seen in mice that received the highest dose suggests the possibility of nausea, and all subsequent studies were hence carried out with a dose of 25 nmol/kg.
**TABLE 3**. Summary of statistical significance levels of dose-dependent fed blood glucose measurements of EG9 exendin-C-POEGMA shown in **FIG. 10a** compared to PBS control. Data were analyzed by repeated measures two-way analysis of variance (ANOVA), followed by *post hoc* Dunnett's test to evaluate individual differences between a treatment and PBS control at each time point (n=3, **P* < 0.05, ***P* < 0.01, ****P* < 0.001 and *****P* <0.0001).

| Time (h) | Dosage (nmol/kg) | | |
|---|---|---|---|
| | 25 | 50 | 85 |
| 1 | | | |
| 4 | **** | **** | **** |
| 8 | ** | ** | *** |
| 24 | **** | **** | **** |
| 48 | *** | *** | *** |
| 72 | | ** | **** |
| 96 | | | * |

To investigate the effect of MW on the glucose regulatory effect of EG9 exendin-C-POEGMA conjugates, native exendin and conjugates of four different MWs (Mₙ = 25.4, 54.6, 97.2 and 155.0 kDa) were tested at a single s.c. injection at 25 nmol/kg mouse body weight. While unmodified exendin was only able to lower blood glucose for 6 h relative to PBS control (**FIG. 3a**, full glucose profiles in **FIG. 11**), modification with EG9 POEGMA significantly extended the glucose-lowering effect of exendin for up to 120 h, with a MW-dependence on the onset, magnitude and duration of the effect (**FIG. 3b****-e, TABLE 4**). As is evident from the overlaid glucose profiles in **FIG. 12a** (overlaid un-normalized glucose profiles in **FIG. 12b**), an increase in MW delays the onset but prolongs the duration of glucose reduction, and the two higher MW conjugates showed an overall smaller magnitude of glucose reduction. This trend is mirrored by the weight profiles of treated animals as well (**FIG. 12c**). The two higher MW conjugates also showed much more flat and steady glucose profiles. The glucose profile of the 155.0 kDa conjugate in particular resembled that of a sustained release depot, with no peak-to-valley effect that can cause undesirable side effects.

**TABLE 4.** Summary of statistical significance levels of MW-dependent fed blood glucose measurements of EG9 exendin-C-POEGMA conjugates shown in **FIG. 3** compared to PBS control. Data were analyzed by repeated measures two-way ANOVA, followed by *post hoc* Dunnett's multiple comparison test to evaluate individual differences between a treatment and PBS control at each time point (n=6, **P* < 0.05, ***P* < 0.01, ****P* < 0.001 and *****P* <0.0001).Groups treated with conjugates were not measured at t = 6 h.

| Time (h) | exendin | EG9 exendin-POEGMA | | | |
|---|---|---|---|---|---|
| | | 25.4 kDa | 54.6 kDa | 97.2 kDa | 155.0 kDa |
| 1 | *** | **** | | | |
| 4 | **** | **** | *** | * | |
| 6 | **** | --- | --- | --- | --- |
| 8 | | **** | **** | ** | * |
| 24 | | **** | **** | **** | **** |
| 48 | | * | **** | **** | **** |
| 72 | | ** | **** | **** | **** |
| 96 | | | *** | *** | **** |
| 120 | | | | ** | |

The *in vitro* cAMP results and the *in vivo* MW-dependent fed glucose measurements collectively show that an increase in MW of the conjugated polymer decreases the potency but increases the circulation duration of the EG9 exendin-POEGMA conjugate. Therefore, we hypothesize that there exists an optimal MW of the conjugate that best balances these two opposing effects. The area under the curve (AUC) of the fed glucose profiles with respect to 0% baseline signifies total glucose exposure, which accounts for both the magnitude and duration of glucose reduction, and is therefore a manifestation of the combined effect of the two opposing factors. Plotting the AUC of fed glucose levels as a function of conjugate Mₙ indeed yielded a roughly inverted bell-shaped distribution with a minimum at 54.6 kDa (**FIG. 3f**). This suggests that the 54.6 kDa conjugate is the optimal among the tested EG9 conjugates in terms of balancing receptor activation potency and sustained duration of action. We thus investigated the 54.6 kDa EG9 conjugate further in subsequent experiments.

To validate the results from the fed glucose measurements and to obtain further evidence of the efficacy of EG9 exendin-C-POEGMA conjugates, an intraperitoneal glucose tolerance test (IPGTT) was performed 24 h and 72 h after a single s.c. injection of the 54.6 kDa EG9 conjugate or unmodified exendin at 25 nmol/kg. IPGTT confirmed the prolonged presence of the conjugate in circulation and its significant effect on glycemic control: at 24 h post-injection, the AUC of blood glucose level over 2 h after glucose challenge is reduced by 68% (P < 0.0001, **FIG. 4a**), and at 72 h post-injection, the AUC is reduced by 48% for conjugate-treated mice compared with PBS controls (P < 0.01, **FIG. 4b**). This is in stark contrast to the unmodified exendin group, which was insignificant at both time points (**FIG. 4c** and **FIG. 4d****)**.

### Example 5

### Antigenicity of EG9 exendin-C-POEGMA conjugates (Reference Example)

We tested the reactivity of the 54.6 kDa EG9 exendin-C-POEGMA conjugate to anti-PEG antibodies in plasma samples of patients previously treated with PEGylated proteins using enzyme-linked immunosorbant assay (ELISA). In a direct ELISA, the 54.6 kDa EG9 exendin-C-POEGMA conjugate and various controls, including two FDA-approved drugs, Adagen^{®}― a PEGylated adenosine deaminase for treating severe combined immunodeficiency disease (SCID) and Krystexxa^{®}― a PEGylated uricase for treating chronic refractory gout, were directly coated on a plate and probed with diluent, an anti-PEG negative patient plasma sample or one of two anti-PEG positive patient plasma samples. As shown in **FIG. 5a**, while the EG9 exendin-C-POGEMA conjugate did show a small amount of binding to anti-PEG antibodies in the positive plasma samples, the extent of binding is significantly less than those of the two PEGylated positive controls. This result was confirmed by a competitive ELISA, where Krystexxa^{®} was coated on wells, and different amounts of 54.6 kDa EG9 exendin-C-POEGMA and controls were added in solution to compete for binding to anti-PEG antibodies in an anti-PEG positive plasma sample. As can be seen in **FIG. 5b**, at all tested competing antigen amounts, 54.6 kDa EG9 exendin-C-POEGMA showed significantly reduced antibody binding compared to the positive control, Adagen^{®}.

### Example 6

### Exendin-C-POEGMA with shorter side-chain length

These results led us to hypothesize that the reduced PEG antigenicity of the EG9 exendin-C-POEGMA conjugate is due to both the branched architecture and the short side-chain length of the conjugated POEGMA. As a minimum length of PEG is presumably needed for antibody recognition and binding, we hypothesized that optimizing the side-chain OEG length may further reduce or possibly eliminate the antigenicity of POEGMA conjugates to anti-PEG antibodies. To test this hypothesis, we next synthesized exendin-C-POEGMA conjugates using OEGMA monomer with precisely 3 EG side-chain repeats as seen by LC/ESI-MS (**FIG. 8b**), as evidence in the literature suggests that the antigenic determinant of PEG may be ~6-7 EG repeats. Three different EG3 exendin-C-POEGMA conjugates with Mₙs of 26.3, 55.6, and 71.6 kDa (**TABLE 1**) were synthesized. Assessment of conjugate potency by intracellular cAMP ELISA (**FIG. 13**) showed that similar to the EG9 conjugates, conjugation of EG3 POEGMA to the C-terminus of exendin caused an increase in the EC₅₀ (**TABLE 1**), indicating a decrease in the receptor activation of the conjugates, though with a less pronounced MW-dependence.

### Example 7

### Antigenicity and efficacy of EG3 exendin-C-POEGMA conjugates

We next tested the reactivity of a 55.6 kDa EG3 exendin-C-POEGMA conjugate to anti-PEG antibodies in patient plasma samples. The 54.6 kDa EG9 conjugate was included as a control to confirm the repeatability of the assays. Remarkably, both direct and competitive anti-PEG ELISAs (**FIG. 5c** and **FIG. 5d**) showed that reducing the side-chain length of the conjugated POEGMA down to 3 EG repeats completely eliminated the reactivity of the conjugate toward anti-PEG antibodies present in the patient plasma samples.

As the OEG side-chains on POEGMA are largely responsible for the "stealth" behavior of the polymer and its conjugates, alteration on the side-chain length can thus have an impact on the *in vivo* behavior of POEGMA conjugates. Therefore, we next investigated the *in vivo* efficacy of EG3 exendin-C-POEGMA. The 55.6 kDa and 71.6 kDa EG3 exendin-C-POEGMA conjugates were administered into fed mice via a single s.c. injection at 25 nmol/kg mouse body weight. As can be seen from the post-injection glucose profiles in **FIG. 6a** and **FIG. 6b** (unnormalized glucose profiles and weight profiles in **FIG. 14**), both conjugates significantly reduced mouse blood glucose for up to 96 h compared to PBS control. The EG3 conjugates appear to have slightly lower magnitudes of glucose reduction and more flat glucose profiles compared to their EG9 counterparts.

### Example 8

### Pharmacokinetics of exendin-C-POEGMA conjugates

To further confirm the prolonged circulation of exendin-C-POEGMA conjugates and to seek some answers to the difference between the glucose profiles of EG9 and EG3 conjugates, a pharmacokinetics study was performed with fluorescently labeled exendin, the 54.6 kDa EG9, 55.6 kDa EG3 and 71.6 kDa EG3 conjugates. Two MWs of the EG3 conjugate were tested, as the EG3 and EG9 conjugates have different Rₕs at the same MW. The MWs were chosen such that the 54.6 kDa EG9 conjugate (Rₕ = 5.4±0.6 nm) has similar MW as the 55.6kDa EG3 conjugate and similar Rₕ as the 71.6 kDa EG3 conjugate (Rₕ = 5.6±0.5 nm). The plasma concentration-time courses (**FIG. 6c** and **FIG. 6d**) were analyzed using a non-compartmental fit characterizing the absorption and elimination phases of the pharmacokinetic profiles, to approximate the parameters shown in **TABLE 2.**

**TABLE 2.** Pharmacokinetic parameters of exendin and exendin-C-POEGMA conjugates injected s.c. derived from data analyzed with a non-compartmental fit in **FIG. 6c** and **FIG. 6d****.** Values are reported as mean ± SEM. t_{1/2 a} : absorption half-life, t_{1/2 el}: elimination half-life, Cₘₐₓ: maximum plasma concentration, tₘₐₓ: time to attain Cₘₐₓ. ^{a}Derived from curve fitting. ^{b}Calculated from t = 0 to ∞ from curve fitting.

| | t_{1/2 a} (h) | t_{1/2 el} (h) | Cₘₐₓ (nM)^{a} | tₘₐₓ (nM)^{a} | AUC (h*nM)^{b} |
|---|---|---|---|---|---|
| exendin | 0.7±0.1 | 1.7±0.2 | 37.1±3.8 | 1.78±0.1 | 217.5±36.5 |
| 54.6 kDa EG9 | 6.2±0.5 | 42.4±2.9 | 56.4±3.9 | 20.1±0.4 | 4,795.5±440.7 |
| 55.6 kDa EG3 | 7.6±0.7 | 61.2±5.0 | 44.0±2.7 | 28.5±2.3 | 4,775.0±482.9 |
| 71.6 kDa EG3 | 9.0±1.7 | 61.5±3.2 | 37.7±5.0 | 32.4±3.9 | 4,411.2±499.6 |

After s.c. injection, unmodified exendin had a very short residence time in circulation, with a rapid absorption phase (t_{1/2a} = 0.7±0.1 h) and a short terminal elimination phase (t_{1/2el} = 1.7±0.2 h). In contrast, the exendin-C-POEGMA conjugates tested increased the absorption time by ~ 9 to 13-fold, with the two EG3 conjugates taking longer than the EG9 conjugate to absorb into circulation. Similarly, the 54.6 kDa EG9 conjugate prolonged the elimination phase of exendin by ~ 25-fold, while the two EG3 conjugates afforded a bigger increase of ~36-fold. These differences in the pharmacokinetics resulted in ~ 20-fold increase in AUC for the conjugates compared to unmodified exendin, indicating that conjugation of POEGMA to the C-terminus of exendin significantly enhanced the cumulative exposure of the peptide in circulation. While the Cₘₐₓ of the two EG3 conjugates were considerably lower than that of the EG9 conjugate, consistent with the lower magnitude of glucose reduction seen for the EG3 conjugates in the fed blood glucose studies (FIG. 6a and FIG. 6b), the AUC of the three tested conjugates were comparable given the longer absorption and elimination half-lives of the EG3 conjugates.

The tests with EG9 conjugates form reference examples.

### Example 9 (Reference Example)

### Macroinitiator characterization

The purified exendin-C-Br macroinitiator was characterized by matrix assisted laser desorption ionization-mass spectrometry (MALDI-MS) to confirm initiator attachment (FIG. 15). A major peak was detected at 5,132.55 Da, which closely agrees with the theoretical mass of 5,131.44 Da corresponding to a single N-(2-(2-(2-(2-aminoacetamido)acet-amido)acetamido) ethyl)-2-bromo-2-methylpropanamide (AEBMP) initiator molecule attached to exendin. To verify the site-specificity of initiator attachment, exendin-C-Br was subjected to trypsin digestion and the peptide fragments were analyzed by liquid chromatography/tandem mass spectrometry (LC-MS/MS). Only the C-terminal peptide fragment was detected as a singly brominated cation and its experimental isotope distribution (**FIG. 16a**) showed nearly perfect overlap with its theoretical distribution (FIG. 16b), proving that a single initiator molecule was attached exclusively to the C-terminus of exendin.

### Example 10

### Characterization of EG3 exendin-C-POEGMA conjugates

Three EG3 exendin-C-POEGMA conjugates of different molecular weights (MWs) were synthesized by varying Atom Transfer Radical Polymerization (ATRP) reaction times. The different MWs of the conjugates are evident from the Size Exclusion Chromatography (SEC) peaks eluting at 17.2, 18.2 and 20.3 min, detected by UV-vis absorbance at 280 nm (FIG. 17a) and refractive index (RI, **FIG. 17b****)**. Integration of peak areas in the UV-vis chromatograms showed that the conjugates constituted -65% of the polymerization products on average. The relatively lower conjugation efficiency of the EG3 conjugates compared to their EG9 counterparts is speculated to be due to the considerably lower water solubility of the EG3 OEGMA monomer, though such a yield is still well above the yield that is typically achieved with conventional PEGylation. The conjugates were purified by a single round of preparative SEC (**FIG. 17c**).

It is to be understood that the phraseology or terminology herein is for the purpose of description and not of limitation, such that the terminology or phraseology of the present specification is to be interpreted by the skilled artisan in light of the teachings and guidance.

The breadth and scope of the present disclosure should not be limited by any of the above-described exemplary aspects, but should be defined only in accordance with the following claims.

### SEQUENCES

SEQ ID NO: 1
   Sortase A recognition site, polypeptide
   LPXTG (where X is any amino acid)
SEQ ID NO: 2
   Sortase A recognition site, polypeptide
   LPETG
SEQ ID NO: 3
   Sortase A recognition site, polypeptide
   LPXZG wherein X and Z are independently any amino acid
SEQ ID NO: 4
   Linker, polypeptide
   (GGC)
SEQ ID NO: 5
   Linker, polypeptide
   (GGC)₈
SEQ ID NO: 6
   Linker, polypeptide
   (G₄S)₃
SEQ ID NO: 7
   Linker, polypeptide
   (VPGXG)₁₆ wherein X is valine or cysteine present in a ratio of 1:1.
SEQ ID NO: 8
   "AEBMP", polypeptide
   NGGPSSGAPPPSLPET

## Claims

1. A method of reducing the antigenicity of a molecule, the method comprising conjugating at least one branched polymer to a molecule to form a molecule-polymer conjugate,
wherein the molecule comprises a polypeptide, a polynucleotide, a small molecule, or a combination thereof,
wherein the branched polymer comprises poly[oligo(ethylene glycol) methyl ether methacrylate] (POEGMA),
wherein the POEGMA comprises:
a backbone comprising poly(methyl methacrylate); and
a plurality of side chains covalently attached to the backbone, where each side chain comprises 2 to 7 monomers of ethylene glycol (EG) repeated in tandem;
wherein the molecule-polymer conjugate is not reactive with pre-existing anti-PEG antibodies in a subject, and
wherein the molecule-polymer conjugate has reduced or eliminated antigenicity compared to a control comprising the molecule conjugated to unbranched PEG.

2. The method of claim 1, wherein the molecule is conjugated to the backbone of the branched polymer.

3. The method of claim 1, wherein each side chain has a first terminal end and a second terminal end, wherein the first terminal end is covalently attached to the backbone, and wherein the second terminal end does not include a hydroxyl group.

4. The method of any one of the previous claims, wherein:
each side chain comprises 3 to 7 monomers of ethylene glycol (EG) repeated in tandem; or
each side chain comprises 3 monomers of ethylene glycol (EG) repeated in tandem.

5. The method of any one of claims 1-4, wherein the branched polymer is synthesized and subsequently grafted to the molecule to form the molecule-polymer conjugate.

6. The method of any one of claims 1-5, wherein the conjugating comprises attaching an initiator agent to the molecule to form a macroinitiator; and incubating the macroinitiator with a monomer under conditions that permit free-radical polymerization and formation of a branched polymer to occur from the initiator agent to form the molecule-polymer conjugate.

7. A molecule-polymer conjugate having reduced or eliminated antigenicity compared to a control, the molecule-polymer conjugate comprising:
a branched polymer comprising poly[oligo(ethylene glycol) methyl ether methacrylate] (POEGMA); and
a molecule conjugated to the backbone of the branched polymer, wherein the molecule comprises a polypeptide, a polynucleotide, a small molecule, or a combination thereof,
wherein
the POEGMA comprises:
a backbone comprising poly(methyl methacrylate); and
a plurality of side chains covalently attached to the backbone, where each side chain comprises 2 to 7 monomers of ethylene glycol (EG) repeated in tandem;
wherein the control comprises the molecule conjugated to unbranched PEG;
and wherein the molecule-polymer conjugate is not reactive with pre-existing anti-PEG antibodies in a subject.

8. The conjugate of claim 7, wherein each side chain has a first terminal end and a second terminal end, wherein the first terminal end is covalently attached to the backbone, and wherein the second terminal end independently comprises an alkyl, ester, amine, amide, or carboxyl group.

9. The conjugate of either claim 7 or claim 8, wherein each side chain has a first terminal end and a second terminal end, wherein the first terminal end is covalently attached to the backbone, and wherein the second terminal end does not include a hydroxyl group.

10. The conjugate of any one of claims 7 to 9, wherein:
each side chain comprises 3 monomers of ethylene glycol (EG) repeated in tandem; or
each side chain comprises 3 to 7 monomers of ethylene glycol (EG) repeated in tandem.

11. The method or conjugate of any one of the preceding claims, wherein the molecule comprises one or more peptides or protein therapeutic agents selected from a monoclonal antibody, blood factor, betatrophin, exendin, enzyme, asparaginase, glutamase, arginase, arginine deaminase, adenosine deaminase (ADA), ADA-2, ribonuclease, cytosine deaminase, trypsin, chymotrypsin, papain, growth factor, epidermal growth factor (EGF), insulin, insulin-like growth factor (IGF), transforming growth factor (TGF), nerve growth factor (NGF), plateletderived growth factor (PDGF), bone morphogenic protein (BMP), fibroblast growth factor (FGF), somatostatin, somatotropin, somatropin, somatrem, calcitonin, parathyroid hormone, colony stimulating factors (CSF), clotting factors, tumor necrosis factors (TNF), gastrointestinal peptides, vasoactive intestinal peptide (VIP), cholecystokinin (CCK), gastrin, secretin, erythropoietins, growth hormone , GRF, vasopressins, octreotide, pancreatic enzymes, superoxide dismutase, thyrotropin releasing hormone (TRH), thyroid stimulating hormone, luteinizing hormone, luteinizing hormone-releasing hormone (LHRH), growth hormone releasing hormone (GHRH), tissue plasminogen activators, interleukins, interleukin-1, interleukin-15, interleukin-2, interleukin-10, colony stimulating factor, granulocyte macrophage colonystimulating factor (GM-CSF), interleukin-1 receptor antagonist (IL-1RA), glucagon-like peptide-1 (GLP-1), exenatide, GLP-1 R multi-agonist, GLP-1 R antagonist, GLP-2, TNF-related apoptosisinducing ligand (TRAIL), leptin, ghrelin, granulocyte monocyte colony stimulating factor (GM-CSF), interferons, interferon-a, interferon-gamma, human growth hormone (hGH) and antagonist, macrophage activator, chorionic gonadotropin, heparin, atrial natriuretic peptide, hemoglobin, relaxin, cyclosporine, oxytocin, vaccines, monoclonal antibodies, single chain antibodies, ankyrin repeat proteins, affibodies, activin receptor 2A extracellular domain, alpha-2 macroglobulin, alpha-melanocyte, apelin, bradykinin B2 receptor antagonist, cytotoxic T-lymphocyte-associated protein (CTLA-4), elafin, Factor IX, Factor VIIa, Factor VIII, hepcidin, infestin-4, kallikrein inhibitor, L4F peptide, lacritin, parathyroid hormone (PTH), peptide YY (PYY), thioredoxin, thymosin B4, urate oxidase, urodilatin, aptamers, silencing RNA, microRNA, long non-coding RNA, ribozymes, and combinations thereof.

## Patentansprüche

1. Verfahren zum Reduzieren der Antigenizität eines Moleküls, wobei das Verfahren das Konjugieren von mindestens einem verzweigten Polymer an ein Molekül zum Bilden eines Molekülpolymerkonjugats umfasst,
wobei das Molekül ein Polypeptid, ein Polynukleotid, ein kleines Molekül oder eine Kombination davon umfasst,
wobei das verzweigte Polymer Poly[oligo(ethylenglykol)methylethermethacrylat] (POEGMA) umfasst,
wobei das POEGMA Folgendes umfasst:
eine Rückgratkette umfassend Poly(methylmethacrylat); und
eine Mehrzahl von Seitenketten, die kovalent an die Rückgratkette angelagert sind, wobei jede Seitenkette 2 bis 7 Monomere von Ethylenglykol (EG) umfasst, die sich hintereinander wiederholen;
wobei das Molekülpolymerkonjugat mit vorbestehenden Anti-PEG-Antikörpern in einem Subjekt nicht reaktiv ist; und
wobei das Molekülpolymerkonjugat reduzierte oder eliminierte Antigenizität im Vergleich mit einer Kontrolle umfassend das Molekül, das an unverzweigtes PEG konjugiert ist, aufweist.

2. Verfahren nach Anspruch 1, wobei das Molekül an die Rückgratkette des verzweigten Polymers konjugiert ist.

3. Verfahren nach Anspruch 1, wobei jede Seitenkette ein erstes terminales Ende und ein zweites terminales Ende aufweist, wobei das erste terminale Ende kovalent an die Rückgratkette angelagert ist und wobei das zweite terminale Ende keine Hydroxylgruppe umfasst.

4. Verfahren nach einem der vorhergehenden Ansprüche, wobei:
jede Seitenkette 3 bis 7 Monomere von Ethylenglykol (EG) umfasst, die sich hintereinander wiederholen; oder
jede Seitenkette 3 Monomere von Ethylenglykol (EG) umfasst, die sich hintereinander wiederholen.

5. Verfahren nach einem der Ansprüche 1-4, wobei das verzweigte Polymer synthetisiert und daraufhin auf das Molekül aufgepfropft wird, um das Molekülpolymerkonjugat zu bilden.

6. Verfahren nach einem der Ansprüche 1-5, wobei das Konjugieren Folgendes umfasst: Anlagern eines Initiatormittels an das Molekül, um einen Makroinitiator zu bilden; und Inkubieren des Makroinitiators mit einem Monomer unter Bedingungen, die gestatten, dass eine Radikalpolymerisation und die Bildung eines verzweigten Polymers aus dem Initiatormittel zum Bilden des Molekülpolymerkonjugats erfolgt.

7. Molekülpolymerkonjugat, das im Vergleich mit einer Kontrolle reduzierte oder eliminierte Antigenizität aufweist, wobei das Molekülpolymerkonjugat Folgendes umfasst:
ein verzweigtes Polymer, das Poly[oligo(ethylenglykol)methylethermethacrylat] (POEGMA) umfasst; und
ein Molekül, das an die Rückgratkette des verzweigten Polymers konjugiert ist, wobei das Molekül ein Polypeptid, ein Polynukleotid, ein kleines Molekül oder eine Kombination davon umfasst,
wobei das POEGMA Folgendes umfasst:
eine Rückgratkette umfassend Poly(methylmethacrylat); und
eine Mehrzahl von Seitenketten, die kovalent an die Rückgratkette angelagert sind, wobei jede Seitenkette 2 bis 7 Monomere von Ethylenglykol (EG) umfasst, die sich hintereinander wiederholen;
wobei die Kontrolle das Molekül umfasst, das an unverzweigtes PEG konjugiert ist;
und wobei das Molekülpolymerkonjugat mit vorbestehenden Anti-PEG-Antikörpern in einem Subjekt nicht reaktiv ist.

8. Konjugat nach Anspruch 7, wobei jede Seitenkette ein erstes terminales Ende und ein zweites terminales Ende aufweist, wobei das erste terminale Ende kovalent an die Rückgratkette angelagert ist und wobei das zweite terminale Ende unabhängig eine Alkyl-, Ester-, Amin-, Amid- oder Carboxylgruppe umfasst.

9. Konjugat nach entweder Anspruch 7 oder Anspruch 8, wobei jede Seitenkette ein erstes terminales Ende und ein zweites terminales Ende aufweist, wobei das erste terminale Ende kovalent an die Rückgratkette angelagert ist und wobei das zweite terminale Ende keine Hydroxylgruppe umfasst.

10. Konjugat nach einem der Ansprüche 7 bis 9, wobei
jede Seitenkette 3 Monomere von Ethylenglykol (EO) umfasst, die sich hintereinander wiederholen; oder
jede Seitenkette 3 bis 7 Monomere von Ethylenglykol (EG) umfasst, die sich hintereinander wiederholen.

11. Verfahren oder Konjugat nach einem der vorhergehenden Ansprüche, wobei das Molekül ein oder mehrere Peptide oder therapeutische Proteinmittel umfasst ausgewählt unter einem monoklonalen Antikörper, Blutfaktor, Betatrophin. Exendin, Enzym, Asparaginase, Glutamase, Arginase, Arginindeaminase, Adenosindeaminase (ADA), ADA-2, Ribonuclease, Cytosindeaminase, Trypsin, Chymotrypsin, Papain, Wachstumsfaktor, epidermalem Wachstumsfaktor (EGF), Insulin, insulinähnlichem Wachstumsfaktor (IGF), Transformierender Wachstumsfaktor (TGF), Nervenwachstumsfaktor (NGF), von Plättchen abgeleitetem Wachstumsfaktor (PDGF), knochenmorphogenetischem Protein (BMP), Fibroblastwachstumsfaktor (FGF), Somatostatin, Somatotropin, Somatropin, Somatrem, Calcitonin, Nebenschilddrüsenhormon, koloniestimulierenden Faktoren (CSF), Gerinnungsfaktoren, Tumornekrosefaktoren (TNF), gastrointestinalen Peptiden, vasoaktivem intestinalem Peptid (VIP), Cholecystokinin (CCK), Gastrin, Secretin, Erythropoetinen, Wachstumshormon, GRF, Vasopressinen, Octreotid, pankreatischen Enzymen, Superoxiddismutase, Thyrotropin freisetzendem Hormon (THR), thyroidstimulierendem Hormon, luteinisierendem Hormon, luteinisierendes Hormon freisetzendem Hormon (LHRH), Wachstumshormon freisetzendem Hormon (GHRH), Gewebeplasminogenaktivatoren, Interleukinen, Interleukin-1, Interleukin-15, Interleukin-2, Interleukin-10, koloniestimulierendem Faktor, Granulozytmakrophagekolonie stimulierendem Faktor (GM-CSF), Interleukin-1-Rezeptorantagonist (IL-1RA), glucagonähnlichem Peptid-1 (GLP-1), Exenatid, GLP-IR-Multiagonist, GLP-1R-Antagonist, GLP-2, mit TNF verbundenem Apoptose induzierendem Ligand (TRAIL), Leptin, Ghrelin, Granulozytmonozytkolonie stimulierendem Faktor (GM-CSF), Interferonen, Interferon-α, Interferon-Gamma, humanem Wachstumshormon (hGA) und - antagonist, Makrophagenaktivator, chorionischem Gonadotropin, Heparin, atrialem natriuretischem Peptid, Hämoglobin, Relaxin, Cyclosporin, Oxytocin, Vakzinen, monoklonalen Antikörpern, Einzelkettenantikörpern, Ankrynwiederholungsproteinen, Affikörpern, extrazellulärer Activinrezeptor 2 A-Domäne, Alpha-2-Makroglobulin, Alpha-Melanozyt, Apelin, Bradykinin B2-Rezeptorantagonist, mit zytotoxischem T-Lymphozyt assoziiertem Protein (CTLA-4), Elafin, Faktor IX, Faktor VIIa, Faktor VIII, Hepcidin, Infestin-4, Kallikreininhibitor, L4F-Peptid, Lacritin, Nebenschilddrüsenhormon (PTH), Peptid YY (PYY), Thioredoxin, Thymosin B4, Uratoxidase, Urodilatin, Aptameren, Silencing RNA, MikroRNA, langer nichtkodierender RNA, Ribozymen und Kombinationen davon.

## Revendications

1. Procédé de réduction de l'antigénicité d'une molécule, le procédé comprenant la conjugaison d'au moins un polymère ramifié à une molécule pour former un conjugué molécule-polymère,
la molécule comprenant un polypeptide, un polynucléotide, une petite molécule, ou une combinaison de ceux-ci,
le polymère ramifié comprenant du poly[méthacrylate d'oligo(éthylène glycol) méthyl éther] (POEGMA),
le POEGMA comprenant :
un squelette comprenant du poly(méthacrylate de méthyle) ; et
une pluralité de chaînes latérales liées de manière covalente au squelette, où chaque chaîne latérale comprend de 2 à 7 monomères d'éthylène glycol (EG) répétés en tandem ;
le conjugué molécule-polymère n'étant pas réactif avec les anticorps anti-PEG préexistants chez un sujet, et
le conjugué molécule-polymère présentant une antigénicité réduite ou éliminée comparé à un témoin comprenant la molécule conjuguée au PEG non ramifié.

2. Procédé selon la revendication 1, la molécule étant conjuguée au squelette du polymère ramifié.

3. Procédé selon la revendication 1, chaque chaîne latérale ayant une première extrémité terminale et une seconde extrémité terminale, la première extrémité terminale étant liée de manière covalente au squelette, et la seconde extrémité terminale n'incluant pas de groupe hydroxyle.

4. Procédé selon l'une quelconque des revendications précédentes :
chaque chaîne latérale comprenant de 3 à 7 monomères d'éthylène glycol (EG) répétés en tandem ; ou
chaque chaîne latérale comprenant 3 monomères d'éthylène glycol (EG) répétés en tandem.

5. Procédé selon l'une quelconque des revendications 1 à 4, le polymère ramifié étant synthétisé et par la suite greffé à la molécule pour former le conjugué molécule-polymère.

6. Procédé selon l'une quelconque des revendications 1 à 5, la conjugaison comprenant la fixation d'un agent initiateur à la molécule pour former un macro-initiateur ; et l'incubation du macro-initiateur avec un monomère sous des conditions permettant que la polymérisation par radicaux libres et la formation d'un polymère ramifié se produisent à partir de l'agent initiateur pour former le conjugué molécule-polymère.

7. Conjugué molécule-polymère ayant une antigénicité réduite ou éliminée comparé à un témoin, le conjugué molécule-polymère comprenant :
un polymère ramifié comprenant du poly[méthacrylate d'oligo(éthylène glycol) méthyl éther] (POEGMA) ; et
une molécule conjuguée au squelette du polymère ramifié, la molécule comprenant un polypeptide, un polynucléotide, une petite molécule, ou une combinaison de ceux-ci,
le POEGMA comprenant :
un squelette comprenant du poly(méthacrylate de méthyle) ; et
une pluralité de chaînes latérales liées de manière covalente au squelette, où chaque chaîne latérale comprend de 2 à 7 monomères d'éthylène glycol (EG) répétés en tandem ;
le témoin comprenant la molécule conjuguée au PEG non ramifié ;
et le conjugué molécule-polymère n'étant pas réactif avec les anticorps anti-PEG préexistants chez un sujet.

8. Conjugué selon la revendication 7, chaque chaîne latérale ayant une première extrémité terminale et une seconde extrémité terminale, la première extrémité terminale étant liée de manière covalente au squelette, et la seconde extrémité terminale comprenant indépendamment un groupe alkyle, ester, aminé, amide, ou carboxyle.

9. Conjugué selon l'une ou l'autre parmi la revendication 7 ou la revendication 8, chaque chaîne latérale ayant une première extrémité terminale et une seconde extrémité terminale, la première extrémité terminale étant liée de manière covalente au squelette, et la seconde extrémité terminale n'incluant pas de groupe hydroxyle.

10. Conjugué selon l'une quelconque des revendications 7 à 9 :
chaque chaîne latérale comprenant 3 monomères d'éthylène glycol (EG) répétés en tandem ; ou
chaque chaîne latérale comprenant de 3 à 7 monomères d'éthylène glycol (EG) répétés en tandem.

11. Procédé ou conjugué selon l'une quelconque des revendications précédentes, la molécule comprenant un ou plusieurs peptides ou agents thérapeutiques protéiques sélectionnés parmi un anticorps monoclonal, un facteur sanguin, la bêtatrophine, l'exendine, une enzyme, l'asparaginase, la glutamase, l'arginase, l'arginine désaminase, l'adénosine désaminase (ADA), l'ADA-2, la ribonucléase, la cytosine désaminase, la trypsine, la chymotrypsine, la papaïne, un facteur de croissance, le facteur de croissance épidermique (EGF), l'insuline, un facteur de croissance type insuline (IGF), un facteur de croissance transformant (TGF), un facteur de croissance des nerfs (NGF), un facteur de croissance dérivé des plaquettes (PDGF), une protéine morphogénique osseuse (BMP), un facteur de croissance des fibroblastes (FGF), la somatostatine, la somatotropine, la somatropine, le somatrem, la calcitonine, l'hormone parathyroïdienne, les facteurs de stimulation de colonie (CSF), les facteurs de coagulation, les facteurs de nécrose tumorale (TNF), les peptides gastro-intestinaux, le peptide intestinal vasoactif (VIP), la cholécystokinine (CCK), la gastrine, la sécrétine, les érythropoïétines, l'hormone de croissance, GRF, les vasopressines, l'octréotide, les enzymes pancréatiques, la superoxyde dismutase, l'hormone de libération de la thyrotropine (TRH), l'hormone de stimulation de la thyroïde, l'hormone lutéinisante, l'hormone de libération d'hormone lutéinisante (LHRH), l'hormone de libération de l'hormone de croissance (GHRH), les activateurs du plasminogène tissulaire, les interleukines, l'interleukine-1, l'interleukine-15, l'interleukine-2, l'interleukine-10, le facteur de stimulation des colonies, le facteur de stimulation des colonies de macrophages granulocytes (GM-CSF), l'antagoniste du récepteur de l'interleukine-1 (IL-1RA), le peptide type glucagon-1 (GLP-1), l'exénatide, le multi-agoniste GLP-1 R, l'antagoniste GLP-1 R, la GLP-2, le ligand induisant l'apoptose liée au TNF (TRAIL), la leptine, la ghréline, le facteur de stimulation des colonies de monocytes granulocytes (GM-CSF), les interférons, l'interféron-a, l'interféron-gamma, l'hormone de croissance humaine (hGH) et l'antagoniste, l'activateur des macrophages, la gonadotropine chorionique, l'héparine, le peptide natriurétique auriculaire, l'hémoglobine, la relaxine, la cyclosporine, l'oxytocine, les vaccins, les anticorps monoclonaux, les anticorps à chaîne unique, les protéines de répétition de l'ankyrine, les afficorps, le domaine extracellulaire 2A du récepteur de l'activine, la macroglobuline alpha-2, l'alphamélanocyte, l'apeline, l'antagoniste du récepteur de la bradykinine B2, la protéine associée aux lymphocytes cytotoxiques T (CTLA-4), l'élafine, le Facteur IX, le Facteur VIIa, le Facteur VIII, l'hepcidine, l'infestine-4, l'inhibiteur de la kallikréine, le peptide L4F, la lacritine, l'hormone parathyroïdienne (PTH), le peptide YY (PYY), la thiorédoxine, la thymosine B4, l'urate oxydase, l'urodilatine, les aptamères, l'ARN de silençage, l'ARNmicro, l'ARN non codant long, les ribozymes, et leurs combinaisons.


### SEQUENCE LISTING

<110> DUKE UNIVERSITY
<120> POLYMER CONJUGATES HAVING REDUCED ANTIGENICITY AND METHODS OF USING THE SAME
<130> 028193-9235-WO00
<140> PCT/US2016/068141
   <141> 2016-12-21
<150> 62/407,403
   <151> 2016-10-12
<150> 62/329,800
   <151> 2016-04-29
<150> 62/310,534
   <151> 2016-03-18
<150> 62/270,401
   <151> 2015-12-21
<160> 8
<170> PatentIn version 3.5
<210> 1
   <211> 5
   <212> PRT
   <213> Sortase A recognition site, polypeptide
<220>
   <221> X
   <222> (3)..(3)
   <223> any amino acid
<400> 1
   Leu Pro Xaa Thr Gly
   1               5
<210> 2
   <211> 5
   <212> **PRT**
   <213> Sortase A recognition site, polypeptide
<400> 2
   Leu Pro Glu Thr Gly
   1               5
<210> 3
   <211> 5
   <212> **PRT**
   <213> Sortase A recognition site, polypeptide
<220>
   <221> X
   <222> (3)..(3)
   <223> any amino acid independent of X located at position 4
<220>
   <221> X
   <222> (4)..(4)
   <223> any amino acid independent of X located at position 3
<400> 3
   Leu Pro Xaa Xaa Gly
   1               5
<210> 4
   <211> 3
   <212> **PRT**
   <213> Linker, polypeptide
<400> 4
   Gly Gly Cys
   1
<210> 5
   <211> 24
   <212> **PRT**
   <213> Linker, polypeptide
<400> 5
   Gly Gly Cys Gly Gly Cys Gly Gly Cys Gly Gly Cys Gly Gly Cys Gly
   1               5                   10                  15
   Gly Cys Gly Gly Cys Gly Gly Cys
               20
<210> 6
   <211> 15
   <212> **PRT**
   <213> Linker, polypeptide
<400> 6
   Gly Gly Gly Gly Ser Gly Gly Gly Gly Ser Gly Gly Gly Gly Ser
   1               5                   10                  15
<210> 7
   <211> 80
   <212> PRT
   <213> Linker, polypeptide
<220>
   <221> X
   <222> (4)..(4)
   <223> valine or cysteine present in a ratio of 1:1
<220>
   <221> X
   <222> (9)..(9)
   <223> valine or cysteine present in a ratio of 1:1
<220>
   <221> X
   <222> (14)..(14)
   <223> valine or cysteine present in a ratio of 1:1
<220>
   <221> X
   <222> (19)..(19)
   <223> valine or cysteine present in a ratio of 1:1
<220>
   <221> X
   <222> (24)..(24)
   <223> valine or cysteine present in a ratio of 1:1
<220>
   <221> X
   <222> (29)..(29)
   <223> valine or cysteine present in a ratio of 1:1
<220>
   <221> X
   <222> (34)..(34)
   <223> valine or cysteine present in a ratio of 1:1
<220>
   <221> X
   <222> (39)..(39)
   <223> valine or cysteine present in a ratio of 1:1
<220>
   <221> X
   <222> (44)..(44)
   <223> valine or cysteine present in a ratio of 1:1
<220>
   <221> X
   <222> (49)..(49)
   <223> valine or cysteine present in a ratio of 1:1
<220>
   <221> X
   <222> (54)..(54)
   <223> valine or cysteine present in a ratio of 1:1
<220>
   <221> X
   <222> (59)..(59)
   <223> valine or cysteine present in a ratio of 1:1
<220>
   <221> X
   <222> (64)..(64)
   <223> valine or cysteine present in a ratio of 1:1
<220>
   <221> X
   <222> (69)..(69)
   <223> valine or cysteine present in a ratio of 1:1
<220>
   <221> X
   <222> (74)..(74)
   <223> valine or cysteine present in a ratio of 1:1
<220>
   <221> X
   <222> (79).. (79)
   <223> valine or cysteine present in a ratio of 1:1
<400> 7
   Val Pro Gly Xaa Gly Val Pro Gly Xaa Gly Val Pro Gly Xaa Gly Val
   1               5                   10                  15
   Pro Gly Xaa Gly Val Pro Gly Xaa Gly Val Pro Gly Xaa Gly Val Pro
               20                  25                  30
   Gly Xaa Gly Val Pro Gly Xaa Gly Val Pro Gly Xaa Gly Val Pro Gly
           35                  40                  45
   Xaa Gly Val Pro Gly Xaa Gly Val Pro Gly Xaa Gly Val Pro Gly Xaa
       50                  55                  60
   Gly Val Pro Gly Xaa Gly Val Pro Gly Xaa Gly Val Pro Gly Xaa Gly
   65                  70                  75                  80
<210> 8
   <211> 16
   <212> **PRT**
   <213> "AEBMP", polypeptide
<400> 8
   Asn Gly Gly Pro Ser Ser Gly Ala Pro Pro Pro Ser Leu Pro Glu Thr
   1               5                   10                  15